# FASCICULE DE BREVET EUROPEEN

(11) **EP 4 308 716 B1**
(45) Date de publication et mention de la délivrance du brevet: **13.05.2026**
(21) Numéro de dépôt: 22715348.3
(22) Date de dépôt: 18.03.2022
(51) Int. Cl.: C12P 13/10

(54) **MOLÉCULE HYBRIDE COMPRENANT UN PEPTIDE CITRULLINÉ DÉRIVÉ DE LA FIBRINE ET UN ANTICORPS OU FRAGMENT D'ANTICORPS SE LIANT À CD38 ET/OU CD138, ET SES UTILISATIONS**
HYBRIDMOLEKÜL MIT EINEM CITRULLINIERTEN PEPTID AUS FIBRIN UND EINEM ANTIKÖRPER ODER ANTIKÖRPERFRAGMENT MIT BINDUNG AN CD38 UND/ODER CD138- SOWIE VERWENDUNGEN DAVON
HYBRID MOLECULE COMPRISING A FIBRIN-DERIVED CITRULLINATED PEPTIDE AND AN ANTIBODY OR ANTIBODY FRAGMENT WHICH BINDS TO CD38 AND/OR CD138, AND USES THEREOF

(30) Priorité: 19.03.2021 FR 2102804
(43) Date de publication de la demande: 24.01.2024
(73) Titulaire: Université Paul Sabatier Toulouse III, 31400 Toulouse (FR); Institut National de la Santé et de la Recherche Médicale (INSERM), 75013 Paris (FR); Centre Hospitalier Universitaire de Toulouse, 31300 Toulouse (FR); Centre Hospitalier Universitaire de Montpellier, 34090 Montpellier (FR); UNIVERSITE DE MONTPELLIER, 34090 Montpellier (FR); Arthritis Recherche & Developpement, 92200 Neuilly sur Seine (FR)
(72) Inventeur: VACHIN, Pauline, 34000 MONTPELLIER (FR); LOUIS-PLENCE, Pascale, 34270 SAINT MATHIEU DE TREVIERS (FR); JORGENSEN, Christian, 34980 SAINT-CLEMENT DE RIVIERE (FR); ESQUERRE, Camille, 31650 AUZIELLE (FR); CLAVEL, Cyril, 31470 SAINT FOY DE PEYROLIERES (FR); SERRE, Guy, Bruno, René, 31100 TOULOUSE (FR); COMBES, Eve, 34560 VILLEVEYRAC (FR); ROBERT, Bruno, 34298 MONTPELLIER CEDEX 5 (FR); MARTINEAU, Pierre, 34090 MONTPELLIER (FR)
(74) Mandataire: Plasseraud IP
(86) Numéro de dépôt international: PCT/FR2022/050505
(87) Numéro de publication internationale: WO 2022/195238

(56) Documents cités:
- EP-B1- 2 176 298
- US-A1- 2009 042 291
- ALETAHA DANIEL: "Precision medicine and management of rheumatoid arthritis", vol. 110, 1 June 2020 (2020-06-01), GB, pages 102405, XP055874103, ISSN: 0896-8411, Retrieved from the Internet <URL:https://www.sciencedirect.com/science/article/pii/S0896841120300068/pdfft?md5=20219f055e5f236f398cab5ef34dc309&pid=1-s2.0-S0896841120300068-main.pdf> DOI: 10.1016/j.jaut.2020.102405
- ANTONIO MANZO ET AL: "Secondary and ectopic lymphoid tissue responses in rheumatoid arthritis: from inflammation to autoimmunity and tissue damage/remodeling", IMMUNOLOGICAL REVIEWS, vol. 233, no. 1, 1 January 2010 (2010-01-01), US, pages 267 - 285, XP055218423, ISSN: 0105-2896, DOI: 10.1111/j.0105-2896.2009.00861.x
- WU CHAO-YI ET AL: "Anti-Citrullinated Protein Antibodies in Patients with Rheumatoid Arthritis: Biological Effects and Mechanisms of Immunopathogenesis", INTERNATIONAL JOURNAL OF MOLECULAR SCIENCES, vol. 21, no. 11, 1 January 2020 (2020-01-01), Basel, CH, pages 4015, XP055873913, ISSN: 1661-6596, DOI: 10.3390/ijms21114015
- DAMIANA TYRILLSHALL ET AL: "Citrullination of fibrinogen by peptidylarginine deiminase 2 impairs fibrin clot structure", CLINICA CHIMICA ACTA, ELSEVIER BV, AMSTERDAM, NL, vol. 501, 12 November 2019 (2019-11-12), pages 6 - 11, XP085994478, ISSN: 0009-8981, [retrieved on 20191112], DOI: 10.1016/J.CCA.2019.10.033
- VARIOUS: "Meeting Abstracts 23rd European workshop for rheumatology research", ARTHRITIS RESEARCH AND THERAPY, BIOMED CENTRAL, LONDON, GB, vol. 5, no. Suppl.1, 27 February 2003 (2003-02-27), pages S1 - S35, XP002293752, ISSN: 1478-6354, DOI: 10.1186/AR1010

## Description

La présente invention concerne une molécule hybride comprenant un peptide citrulliné dérivé de la fibrine et un anticorps ou fragment d'anticorps se liant à CD38 et/ou CD138, les utilisations d'une telle molécule hybride, ainsi que son procédé de production.

### Contexte de l'invention

La polyarthrite rhumatoïde est le plus fréquent des rhumatismes inflammatoires ou arthrites, mais aussi la plus fréquente des maladies auto-immunes. La maladie est caractérisée par une inflammation chronique des articulations synoviales aboutissant à une destruction articulaire irréversible.

La présence d'auto-anticorps de classe G dirigés contre les protéines citrullinées et appelés auto-anticorps anti-protéines citrullinées (ACPA), est hautement spécifique de la polyarthrite rhumatoïde. Les sérums de patients qui contiennent des ACPA, les cellules lymphocytaires qui les expriment à leur membrane et les patients eux-mêmes, sont dits 'ACPA-positifs'. Plusieurs études ont démontré que ces ACPA sont au cœur des réactions auto-immunes propres à la maladie rhumatoïde, et représentent ainsi une cible thérapeutique de choix.

Les cibles antigéniques des ACPA ont été caractérisées. Ils sont notamment dirigés spécifiquement contre des formes désiminées ou citrullinées des chaînes polypeptidiques α et β de la fibrine, protéine abondante dans le tissu synovial inflammatoire. Cette citrullination correspond à la désimination enzymatique des résidus arginyl d'une protéine, sous l'action de peptidyl-arginine désiminases (PAD).

Plus spécifiquement, les épitopes immunodominants reconnus par les ACPA sur les chaînes polypeptidiques α et β de la fibrine ont été caractérisés et publiés, notamment dans les demandes PCT/FR00/01857 ou PCT/FR2007/000758. Les cinq peptides citrullinés porteurs des épitopes immunodominants sont plus particulièrement les peptides nommés α36-50Cit (tel que représenté en SEQ ID NO : 5), α171-185Cit (tel que représenté en SEQ ID NO : 6), α501-515Cit (tel que représenté en SEQ ID NO : 14), α621-635Cit (tel que représenté en SEQ ID NO : 18) et β60-74Cit (tel que représenté en SEQ ID NO : 19). Les sérums de patients ACPA-positifs reconnaissent un ou plusieurs de ces cinq peptides.

Sécrétés dans le tissu synovial rhumatoïde par des plasmocytes locaux, les ACPA y présentent une concentration élevée, à proximité de leur principale cible, la fibrine citrullinée, qui y est, elle aussi, abondante sous forme de dépôts interstitiels. La fixation des ACPA sur ces dépôts et donc la formation de complexes immuns immobilisés, fixant à leur tour les facteurs rhumatoïdes, autre auto-anticorps associé à la polyarthrite rhumatoïde et eux aussi sécrétés par des plasmocytes locaux, déclenche une cascade d'évènements pro-inflammatoires.

La stimulation de cellules macrophagiques par ces macro-complexes immuns, essentiellement via leurs récepteurs Fcgamma membranaires, les conduit à sécréter des cytokines pro-inflammatoires et notamment du TNF-alpha qui a été identifié comme la principale cytokine responsable de l'inflammation rhumatoïde.

A ce jour, il n'existe pas de traitement curatif de la polyarthrite rhumatoïde. Les traitements visent uniquement à soigner les poussées et/ou prévenir leur apparition.

Un objet de la présente invention est ainsi de fournir un traitement à la polyarthrite rhumatoïde.

Les ACPA sont oligoclonaux et donc sécrétés par seulement quelques clones plasmocytaires, résultant eux-mêmes de la différenciation de quelques clones lymphocytaires B.

En cas de polyarthrite rhumatoïde, des clones de lymphocytes B expriment à leur membrane des immunoglobulines porteuses de la spécificité ACPA (il s'agit de lymphocytes B ACPA-positifs), alors que les plasmocytes issus de la différenciation des clones lymphocytaires B ACPA-positifs (il s'agit de plasmocytes ACPA-positifs) sécrètent en abondance des ACPA dans leur microenvironnement.

La présente invention repose sur les résultats des Inventeurs montrant qu'il est possible de cibler les plasmocytes sécrétant en abondance les ACPA dans leur microenvironnement et les éliminer à l'aide d'une molécule hybride comprenant (i) un peptide dérivé de fibrine ayant au moins un résidu citrullyl et (ii) un anticorps ou fragment d'anticorps se liant à CD38 et/ou CD138. Ces molécules hybrides cibleront les plasmocytes (à l'aide de l'anticorps ou fragment d'anticorps se liant à CD38 et/ou CD138 qui sont des marqueurs moléculaires exprimés en surface desdits plasmocytes) et, grâce au peptide dérivé de fibrine ayant au moins un résidu citrullyl, la molécule hybride sera reconnue spécifiquement par les ACPA (le peptide étant la cible de ces ACPA). Après pontage (fixation des ACPA à la molécule hybride, elle-même liée aux plasmocytes), les plasmocytes seront lysés par apoptose, par phagocytose en présence de macrophages, par ADCC (cytotoxicité à médiation cellulaire dépendante des anticorps) en présence de cellules NK, ou encore par activation du complément.

En ciblant les plasmocytes qui sécrètent des ACPA, les molécules hybrides de l'invention visent ainsi à faire disparaître les ACPA de l'organisme des patients et la source de ces ACPA pour induire une rémission de la maladie.

Le ciblage des plasmocytes et des anticorps libérés par lesdits plasmocytes à l'aide d'hybride a déjà été décrit, par exemple dans la demande EP2892926. Cependant, aucune construction spécifique ou résultat relatif à la polyarthrite rhumatoïde n'est décrit.

### Exposé de l'invention

### Molécule hybride selon l'invention

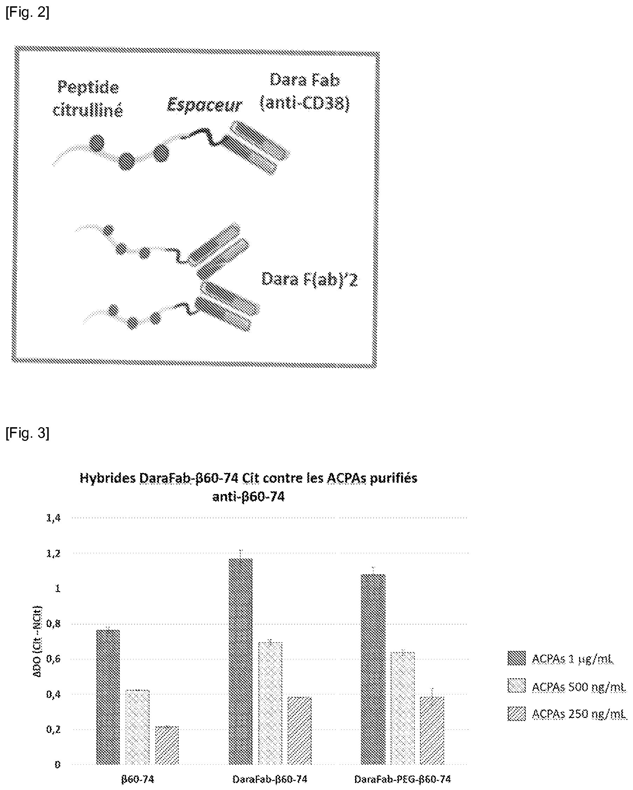

Dans un premier aspect, l'invention concerne une molécule hybride comprenant au moins un peptide dérivé de fibrine ayant au moins un résidu citrullyl, ledit peptide étant lié de façon covalente à au moins un anticorps ou à au moins un fragment d'anticorps, ledit anticorps ou fragment étant capable de se lier à CD38 et/ou CD138, un ou plusieurs espaceurs étant optionnellement présents entre ledit peptide et ledit anticorps ou ledit fragment, ledit peptide étant reconnu par les auto-anticorps anti-protéines citrullinées et étant dérivé de tout ou partie de la séquence de la chaine α ou β d'une fibrine de vertébré, par substitution d'au moins un résidu arginyl par un résidu citrullyl. Le schéma d'une telle construction est présenté en Figure 2.

Selon l'invention, une « molécule hybride » s'entend d'une molécule ayant au moins deux composants de nature différente, en l'espèce ledit peptide et un anticorps ou fragment d'anticorps capable de se lier à CD38 et/ou CD138.

Selon l'invention, un « anticorps » s'entend d'une immunoglobuline. Les immunoglobulines sont constituées d'un assemblage de deux dimères constitués chacun d'une chaîne lourde et d'une chaîne légère. Chacune des chaînes lourdes et des chaînes légères, est constituée d'une région constante et d'une région variable. Plus précisément, chaque chaîne légère est constituée d'une région variable (VL) et d'une région constante (CL). Chaque chaîne lourde est constituée d'une région variable (VH) et d'une région constante constituée de trois ou quatre domaines constants CH1, CH2, CH3 et éventuellement CH4. L'anticorps s'entend des IgM, IgD, IgG, IgA et IgE. De préférence, l'anticorps est une IgG, plus particulièrement IgG1. Un anticorps selon l'invention s'entend également d'un anticorps bispécifique, ayant une affinité pour deux antigènes différents. L'anticorps utilisé dans la molécule hybride selon l'invention est de préférence un anticorps monoclonal.

Selon l'invention, un « fragment anticorps » s'entend d'une portion d'anticorps qui est porteuse du site de liaison à l'antigène. De tels fragments d'anticorps sont par exemple Fab, F(ab')2, Fv, dsFv, scFv, les fragments simples chaines tel que les fragments VH ou VL isolés, les VHH de camélidés, les VNAR de poisson cartilagineux, ou encore des anticorps multispécifiques composés de différents fragments, tels que des anticorps bi-spécifiques, ou encore des « nanobodies » « diabodies », « triabodies » ou « tetrabodies » ou des scFv en tandem. Ces fragments sont bien connus de l'homme du métier. De plus amples informations concernant ces fragments et constructions sont par exemple décrites dans la demande internationale WO2017137579, Bird et al., 1988 Science 242:423-426; and Huston et al., 1988 Proc. Natl. Acad. Sci. 85:5879-5883, ou encore Nelson, mAbs 2:1, 77-83; January/February 2010.

Typiquement, un « Fab » est un fragment d'anticorps qui comprend environ la moitié du côté N-terminal de la chaîne lourde et la chaîne légère entière d'un anticorps, liées par un pont disulfure. Le Fab peut être notamment obtenu à partir d'une IgG par le traitement par une protéase, la papaïne. Un « F(ab')2 » désigne ainsi deux fragments Fab tels que définis ci-dessus, liés entre eux par un pont disulfure. Le F(ab')2 peut être notamment obtenu à partir d'une IgG par le traitement par une protéase, la pepsine.

Typiquement, un « Fv » correspond à deux domaines VL et VH associés de manière non-covalente et un « dsFv » correspond à l'association d'un VH et VL liés par un pont disulfure.

Typiquement, un « scFv » (single chain Fv) est un polypeptide VH:VL synthétisé en utilisant les gènes codant pour les domaines VL et VH et une séquence codant pour un peptide destiné à lier ces domaines. Les scFv peuvent être associés entre eux et former par exemple des diabodies ... (2 scFv), des triabodies (3 scFv) ou des tetrabodies (4 scFv). Dans de telles constructions, les scFv sont généralement liés entre eux par une ou plusieurs liaisons peptidiques. La spécificité antigénique de chaque scFv impliqué dans de telles constructions multimériques peut être identique ou différente.

Selon un mode de réalisation préféré, dans ladite molécule hybride, ledit fragment est choisi parmi les fragments, scFv, Fv, Fab ou F(ab')2, plus particulièrement Fab ou F(ab')2.

Selon l'invention « CD38 » et « CD138 » font référence aux marqueurs exprimés à la surface des plasmocytes. CD138 est spécifique des plasmocytes. CD38 n'est pas spécifique des plasmocytes, mais les plasmocytes sont néanmoins parmi les cellules exprimant le plus fortement ce marqueur. Daratumumab est un exemple d'anticorps thérapeutique ciblant CD38. De préférence, ledit anticorps ou ledit fragment est dirigé contre CD38.

Selon un autre mode de réalisation, dans ladite molécule hybride, ledit anticorps ou ledit fragment est bispécifique, et est dirigé contre : CD38 et une autre cible plasmocytaire ou CD138 et une autre cible plasmocytaire ou CD38 et CD138. Plus particulièrement, selon un mode de réalisation, dans ladite molécule hybride, ledit anticorps ou fragment F(ab')2 est un anticorps ou fragment F(ab')2 bispécifique, dirigé contre :
- CD38 et une autre cible plasmocytaire, ou
- CD138 et une autre cible plasmocytaire, ou
- CD38 et CD138.

Selon l'invention, une « autre cible plasmocytaire » s'entend de tout marqueur étant exprimé à la surface des plasmocytes.

Selon l'invention, l'expression « lié de façon covalente » s'entend d'une liaison covalente, c'est-à-dire une liaison chimique dans laquelle deux atomes se partagent deux électrons. Ladite liaison covalente peut être polaire ou non-polaire.

Selon l'invention, un « espaceur » est un agent de liaison qui permet de lier de façon covalente ledit anticorps ou fragment d'anticorps audit peptide dérivé de fibrine ayant au moins un résidu citrullyl, tout en éloignant ledit anticorps ou fragment dudit peptide (diminuant ainsi un éventuel encombrement stérique). Il peut s'agir de toute molécule, et notamment d'un peptide. De manière préférée, l'espaceur ne modifie pas les propriétés physico-chimiques de la molécule hybride.

La présence d'au moins un espaceur est avantageuse : elle permet de faciliter l'accessibilité indépendante des deux partenaires de la molécule hybride (l'anticorps ou le fragment d'anticorps est plus facilement accessible pour se lier aux plasmocytes, de même que ledit peptide est plus facilement accessible pour se lier aux cellules B ACPA-positives), et/ou de stabiliser la molécule hybride, et/ou d'augmenter la solubilité de la molécule hybride.

Selon un mode de réalisation, la molécule hybride selon l'invention peut comprendre un ou plusieurs espaceurs. De préférence, la molécule hybride comprend un ou deux espaceurs. Selon un mode de réalisation, lorsqu'au moins un espaceur est présent dans ladite molécule hybride de l'invention, ledit anticorps ou fragment d'anticorps est lié de façon covalente à un espaceur, ledit espaceur étant lui-même lié de façon covalente audit peptide. Selon un autre mode de réalisation, lorsqu'au moins deux espaceurs sont présents dans ladite molécule hybride de l'invention, ledit anticorps ou fragment d'anticorps est lié de façon covalente à un premier espaceur, ledit premier espaceur étant lui-même lié de façon covalente à un deuxième espaceur et le deuxième espaceur est lui-même lié de façon covalente audit peptide. La liaison entre l'anticorps ou le fragment d'anticorps et le peptide peut donc être directe, ou bien indirecte en présence d'espaceurs.

Selon un mode de réalisation, la molécule hybride selon l'invention peut comprendre au moins un peptide dérivé de fibrine ayant au moins un résidu citrullyl. Cela signifie que l'anticorps ou le fragment d'anticorps peut être lié à un ou deux peptides. En effet, l'anticorps ou le fragment d'anticorps comprend deux monomères, et à ce titre l'anticorps ou le fragment d'anticorps peut ainsi être lié de façon covalente à un peptide sur un seul des deux monomères, ou bien l'anticorps ou le fragment d'anticorps peut être lié de façon covalente à un peptide sur chaque monomère (voir trois ou quatre en cas d'anticorps ou de fragment bispécifique). De préférence, lorsque deux peptides sont liés sur l'anticorps ou le fragment d'anticorps, les deux peptides sont identiques, par exemple deux peptides de SEQ ID NO : 18 ou SEQ ID NO : 19.

Selon un mode de réalisation, ledit espaceur est un polymère contenant un ou plusieurs motifs de répétition contenant le groupe éther. Selon un mode de réalisation particulier, ledit espaceur est le polyéthylène glycol de formule PEGn, dans laquelle n représente un nombre entier compris entre 1 et 100, préférentiellement entre 1 et 10 et notamment 1, 2, 3, 4 ou 8. Selon l'invention ledit polyéthylène glycol peut être fonctionnalisé, par exemple avec un groupement amine (PEGn-amine tel que PEG-NH₂). Selon l'invention « un nombre entier compris entre 1 et 100 » représente toutes les valeurs entières comprises entre 1 et 100, i.e. ; 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 et 100.

Selon l'invention, l'expression « peptide dérivé de fibrine ayant au moins un résidu citrullyl » s'entend d'une molécule de fibrine ou de fibrinogène dans laquelle au moins un résidu arginyl a été substitué par un résidu citrullyl. Un « peptide dérivé de fibrine ayant au moins un résidu citrullyl » selon l'invention s'entend d'un peptide reconnu par les ACPA, et peut également être nommé un « peptide citrulliné ». De tels peptides peuvent être obtenus à partir de fragments de fibrine ou de fibrinogène naturels, recombinants ou de synthèse. De tels peptides peuvent également être directement synthétisés. Les acides aminés constituant le peptide peuvent être de série L ou D, de préférence de série L. Ladite substitution peut par exemple être réalisée par une étape de désimination enzymatique sous l'action de peptidyl-arginine désiminases (PAD). Un tel peptide peut aussi être obtenu en incorporant directement un ou plusieurs résidus citrullyl dans le peptide synthétisé. Un peptide selon l'invention se lie aux ACPA, et la liaison entre ledit peptide dérivé de fibrine ayant au moins un résidu citrullyl et un ACPA peut être vérifiée à l'aide d'un test ELISA ou tel que décrit dans la publication Sebbag M, Moinard N, Auger I, Clavel C, Arnaud J, Nogueira L, Roudier J, Serre G. Epitopes of human fibrin recognized by the rheumatoid arthritis-specific autoantibodies to citrullinated proteins. Eur J Immunol 36:2250-2263, 2006.

Selon l'invention, ledit peptide est dérivé de tout ou partie de la séquence de la chaine α ou β d'une fibrine de vertébré, par substitution d'au moins un résidu arginyl par un résidu citrullyl. Préférentiellement, ledit peptide est dérivé d'une séquence d'au moins 5 acides aminés consécutifs de la chaine α (notamment représentée par SEQ ID NO : 27) ou β (notamment représentée par SEQ ID NO : 28) d'une fibrine de vertébré. Encore plus particulièrement, ladite fibrine de vertébré est une fibrine de mammifère, de préférence humaine.

Selon un mode de réalisation, dans ladite molécule hybride selon l'invention, le peptide a une taille d'au moins 2 acides aminés consécutifs, 3 acides aminés consécutifs, 4 acides aminés consécutifs, de préférence 5 acides aminés consécutifs, et plus préférentiellement encore entre 5 et 25 acides aminés. Selon l'invention, « entre 5 et 25 » s'entend de toutes les valeurs : 5, 6, 7, 8, 9, 10, 11, 12, 13, 14, 15, 16, 17, 18, 19, 20, 21, 22, 23, 24 et 25. Préférentiellement, ledit peptide a une taille comprise entre 10 et 20 acides aminés, plus particulièrement 15 acides aminés.

Selon un mode de réalisation, dans ladite molécule hybride selon l'invention, ledit peptide est de préférence linéaire.

Selon un mode de réalisation, dans ladite molécule hybride selon l'invention, le peptide peut être modifié de façon à améliorer sa réactivité vis-à-vis des ACPA. A titre d'exemple, les peptides peuvent être cyclisés, les peptides peuvent être de type rétro (les acides aminés de série L sont enchainés selon une séquence inverse de celle du peptide à reproduire), ou de type rétro-inverso (les acides aminés sont de type D au lieu de la série naturelle L et sont enchainés selon une séquence inverse de celle du peptide à reproduire). Selon un mode de réalisation encore plus particulier, dans ladite molécule hybride selon l'invention, la fonction carboxyle (COOH) terminale dudit peptide est remplacée par une fonction carboxamide (CONH₂). De préférence, dans le peptide de SEQ ID NO : 12, la fonction carboxyle (COOH) terminale dudit peptide est remplacée par une fonction carboxamide (CONH₂). A titre d'exemple, le peptide β60-74Cit (tel que représenté en SEQ ID NO : 19) présente avantageusement une telle fonction carboxamide.

Selon un autre mode de réalisation, dans ladite molécule hybride selon l'invention, le peptide peut être modifié de façon à faciliter sa synthèse et/ou améliorer sa stabilité, par exemple par alkylation. Selon un mode de réalisation encore plus particulier, dans ladite molécule hybride selon l'invention, la fonction amine (NH₂) terminale dudit peptide est acétylée. De préférence, dans le peptide de SEQ ID NO : 1, la fonction amine terminale dudit peptide est acétylée. A titre d'exemple, le peptide α621-635Cit (tel que représenté en SEQ ID NO : 18) présente avantageusement une telle fonction acétyl sur son amine (NH₂) terminale.

Selon un mode de réalisation, dans ladite molécule hybride selon l'invention, les fonctions amine et carboxyle du peptide peuvent être sous forme du sel correspondant à l'acide ou à la base.

Selon un mode de réalisation, dans ladite molécule hybride selon l'invention, ledit peptide comprend au moins un résidu citrullyl, et est choisi dans le groupe constitué par :
a) un peptide défini par la séquence X₁PAPPPISGGGYX₂AX₃ (SEQ ID NO : 1) dans laquelle X₁, X₂, et X₃ représentent chacun un résidu citrullyl ou un résidu arginyl, et au moins l'un des résidus X₁ ou X₂ ou X₃ est un résidu citrullyl ;
b) un peptide défini par la séquence GPX₁VVEX₂HQSACKDS (SEQ ID NO : 2) dans laquelle X₁ et X₂ représentent chacun un résidu citrullyl ou un résidu arginyl, et au moins l'un des résidus X₁ ou X₂ est un résidu citrullyl ;
c) un peptide défini par la séquence SGIGTLDGFX₁HX₂HPD (SEQ ID NO : 3) dans laquelle X₁ et X₂ représentent chacun un résidu citrullyl ou un résidu arginyl, et au moins l'un des résidus X₁ ou X₂ est un résidu citrullyl ;
d) un peptide défini par la séquence VDIDIKIX₁SCX₂GSCS (SEQ ID NO : 4) dans laquelle X₁ et X₂ représentent chacun un résidu citrullyl ou un résidu arginyl, et au moins l'un des résidus X₁ ou X₂ est un résidu citrullyl ;
e) un peptide défini par la séquence X₁GHAKSX₂PVX₃GIHTS (SEQ ID NO : 12) dans laquelle X₁, X₂ et X₃ représentent chacun un résidu citrullyl ou un résidu arginyl, et au moins l'un des résidus X₁ ou X₂ ou X₃ est un résidu citrullyl ;
f) un peptide comprenant au moins 5 acides aminés consécutifs, dont au moins un résidu citrullyl, de l'un des peptides a) à e) ci-dessus.

Selon un mode de réalisation particulier, ledit peptide comprend au moins un résidu citrullyl, et est choisi dans le groupe constitué par :
- un peptide défini par la séquence SEQ ID NO : 1 dans laquelle au moins un résidu choisi parmi X₁ ou X₂ ou X₃ est un résidu citrullyl, ou un peptide comprenant un fragment d'au moins 5 acides aminés consécutifs de ladite séquence contenant ledit ou lesdits résidu(s) citrullyl ;
- un peptide défini par la séquence SEQ ID NO : 2 dans laquelle au moins X₁ ou X₂ est un résidu citrullyl, ou un peptide comprenant un fragment d'au moins 5 acides aminés consécutifs de ladite séquence contenant ledit ou lesdits résidu(s) citrullyl ;
- un peptide défini par la séquence SEQ ID NO : 3 dans laquelle au moins X₁ ou X₂ est un résidu citrullyl, ou un peptide comprenant un fragment d'au moins 5 acides aminés consécutifs de ladite séquence contenant ledit ou lesdits résidu(s) citrullyl ;
- un peptide défini par la séquence SEQ ID NO : 4 dans laquelle au moins X₁ ou X₂ est un résidu citrullyl, ou un peptide comprenant un fragment d'au moins 5 acides aminés consécutifs de ladite séquence contenant ledit ou lesdits résidu(s) citrullyl ;
- un peptide défini par la séquence SEQ ID NO : 12 dans laquelle au moins X₁ ou X₂ ou X₃ est un résidu citrullyl, ou un peptide comprenant un fragment d'au moins 5 acides aminés consécutifs de ladite séquence contenant ledit ou lesdits résidu(s) citrullyl.

Selon un mode de réalisation encore plus particulier, dans ladite molécule hybride selon l'invention, ledit peptide comprend au moins un résidu citrullyl, et est choisi dans le groupe constitué par :
- un peptide défini par la séquence SEQ ID NO : 1 dans laquelle X₁, X₂, et X₃ sont des résidus citrullyl, ou un peptide d'au moins 15 acides aminés comprenant ladite séquence (il s'agit d'un peptide de SEQ ID NO : 19) ;
- un peptide défini par la séquence SEQ ID NO : 2 dans laquelle X₁ et X₂ sont des résidus citrullyl, ou un peptide comprenant un fragment d'au moins 5 acides aminés consécutifs de ladite séquence contenant lesdits résidus citrullyl (il s'agit d'un peptide de SEQ ID NO : 5) ;
- un peptide défini par la séquence SEQ ID NO : 3 dans laquelle X₁ et X₂ sont des résidus citrullyl, ou un peptide comprenant un fragment d'au moins 5 acides aminés consécutifs de ladite séquence contenant lesdits résidus citrullyl (il s'agit d'un peptide de SEQ ID NO : 14) ;
- un peptide défini par la séquence SEQ ID NO : 4 dans laquelle X₁ et X₂ sont des résidus citrullyl, ou un peptide comprenant un fragment d'au moins 5 acides aminés consécutifs de ladite séquence contenant lesdits résidus citrullyl (il s'agit d'un peptide de SEQ ID NO : 6) ;
- un peptide défini par la séquence SEQ ID NO : 12 dans laquelle X₁, X₂ et X₃ sont des résidus citrullyl, ou un peptide comprenant un fragment d'au moins 10 acides aminés consécutifs de ladite séquence contenant lesdits résidus citrullyl (il s'agit d'un peptide de SEQ ID NO : 18).

Selon un mode de réalisation encore plus particulier, dans ladite molécule hybride selon l'invention, ledit peptide est choisi dans le groupe constitué par : SEQ ID NO : 5 (α36-50cit_{38,42}), SEQ ID NO : 6 (α171-185cit_{178,181}), SEQ ID NO : 7 (α183-197cit_{186,190}), SEQ ID NO : 8 (α246-260cit₂₅₈), SEQ ID NO : 9 (α259-273cit_{263,271}), SEQ ID NO : 10 (α366-380cit₃₆₇), SEQ ID NO : 11 (α396-410cit₄₀₄), SEQ ID NO : 13 (α411-425cit₄₂₅), SEQ ID NO : 14 (α501-515cit_{510,512}), SEQ ID NO : 15 (α546-560cit₅₄₇), SEQ ID NO : 16 (α561-575cit₅₇₃), SEQ ID NO : 17 (α588-602cit₅₉₁), SEQ ID NO : 18 (α621-635cit_{621,627,630}), SEQ ID NO : 19 (β60-74cit_{60,72,74}), SEQ ID NO : 20 (β210-224cit₂₂₄), SEQ ID NO : 21 (β281-295cit_{285,294}), SEQ ID NO : 22 (β420-434cit₄₂₁) et SEQ ID NO : 23 (β433-447cit_{436,445}), plus particulièrement choisi dans le groupe constitué par : SEQ ID NO : 5 (α36-50cit_{38,42}), SEQ ID NO : 6 (α171-185cit_{178,181}), SEQ ID NO : 14 (α501-515cit_{510,512}), SEQ ID NO : 18 (α621-635cit_{621,627,630}) et SEQ ID NO : 19 (β60-74cit_{60,72,74}), encore plus particulièrement choisi parmi SEQ ID NO : 18 (α621-635cit_{621,627,630}) et SEQ ID NO : 19 (β60-74cit_{60,72,74}).

L'anticorps ou le fragment d'anticorps et le peptide ont chacun des extrémités N- et Cterminale. L'anticorps ou le fragment d'anticorps peut donc être lié via son extrémité N- ou Cterminale à l'extrémité N- ou Cterminale du peptide. Selon un autre mode de réalisation préféré, dans ladite molécule hybride selon l'invention ladite liaison covalente se situe entre l'extrémité Nterminale dudit peptide et l'extrémité Nterminale dudit anticorps ou fragment d'anticorps, ou entre l'extrémité Nterminale dudit peptide et l'extrémité Cterminale dudit anticorps ou fragment d'anticorps. Selon un mode de réalisation, lorsqu'un espaceur est présent, l'espaceur peut être lié à l'anticorps ou au fragment via son extrémité N- ou Cterminale. Selon un autre mode de réalisation, lorsque deux espaceurs sont présents, le premier espaceur peut être lié à l'anticorps ou le fragment d'anticorps via son extrémité N- ou Cterminale et le deuxième espaceur peut être lié au peptide via son extrémité N- ou Cterminale, notamment Nterminale. Alternativement, ladite liaison covalente entre ledit anticorps ou ledit fragment et ledit peptide (éventuellement en présence d'un ou plusieurs espaceurs) peut être créée sur tout ou partie de l'anticorps ou fragment, sous réserve que celui-ci conserve sa capacité de liaison aux plasmocytes. Selon l'invention « tout ou partie de l'anticorps ou du fragment » signifie que différents acides aminés constituant l'anticorps ou le fragment peuvent être impliqués dans une liaison covalente avec ledit peptide.

Selon un mode de réalisation préféré, lorsqu'au moins un espaceur est présent dans ladite molécule hybride de l'invention, celui-ci permet de lier l'anticorps ou le fragment à un azoture ou à un alcyne qui sera lui-même impliqué dans la liaison covalente avec ledit peptide. Selon un mode de réalisation, lorsqu'au moins un espaceur est présent dans ladite molécule hybride de l'invention, celui-ci peut également permettre de lier le peptide à un alcyne ou à un azoture qui sera lui-même impliqué dans la liaison covalente avec l'anticorps ou le fragment. Selon un mode de réalisation encore plus particulier, la molécule hybride selon l'invention comprend au moins deux espaceurs : un premier espaceur qui permet de lier l'anticorps ou le fragment à un azoture ou à un alcyne et un deuxième espaceur qui permet de lier le peptide à un azoture (lorsque l'anticorps ou le fragment est lié à un alcyne) ou à un alcyne (lorsque le fragment Fc est lié à un azoture).

Selon un mode de réalisation selon l'invention, dans ladite molécule hybride selon l'invention, ledit anticorps ou ledit fragment :
- est couplé à un azoture ou un alcyne tel qu'un cyclooctyne, et en particulier le DBCO, ou
- est lié à un espaceur qui est lui-même couplé à un azoture ou à un alcyne, tel qu'un cyclooctyne, et en particulier le DBCO, et ledit peptide est :
- soit couplé à un azoture ou à un alcyne, tel qu'un cyclooctyne, et en particulier le DBCO,
- soit lié à un espaceur qui est lui-même couplé à un azoture ou à un alcyne, tel qu'un cyclooctyne, et en particulier le DBCO,
la liaison covalente entre ledit fragment et ledit peptide, éventuellement en présence d'un ou plusieurs espaceurs, ou étant créée entre l'azoture et l'alcyne.

Selon un mode de réalisation selon l'invention, dans ladite molécule hybride selon l'invention :
- ledit anticorps ou ledit fragment est couplé à un azoture et ledit peptide est couplé à un alcyne tel qu'un cyclooctyne, et en particulier le DBCO, ou
- ledit anticorps ou ledit fragment est couplé à un alcyne tel qu'un cyclooctyne, et en particulier le DBCO, et ledit peptide est couplé à un azoture,
la liaison covalente entre ledit anticorps ou ledit fragment et ledit peptide étant créée entre l'azoture et l'alcyne.

Selon un mode de réalisation selon l'invention, dans ladite molécule hybride selon l'invention :
- ledit anticorps ou ledit fragment est lié à un espaceur, lui-même couplé à un azoture, et ledit peptide est couplé à un alcyne tel qu'un cyclooctyne, et en particulier le DBCO, ou
- ledit anticorps ou ledit fragment est lié à un espaceur, lui-même couplé à un alcyne tel qu'un cyclooctyne, et en particulier le DBCO, et ledit peptide est couplé à un azoture,
la liaison covalente entre ledit anticorps ou ledit fragment et ledit peptide, en présence d'un espaceur, étant créée entre l'azoture et l'alcyne.

Selon un mode de réalisation selon l'invention, dans ladite molécule hybride selon l'invention :
- ledit anticorps ou ledit fragment est lié à un espaceur, lui-même couplé à un azoture, et ledit peptide est également lié à un deuxième espaceur, lui-même couplé à un alcyne tel qu'un cyclooctyne, et en particulier le DBCO, ou
- ledit anticorps ou ledit fragment est lié à un espaceur, lui-même couplé à un alcyne tel qu'un cyclooctyne, et en particulier le DBCO, et ledit peptide est également lié à un deuxième espaceur, lui-même couplé à un azoture,
la liaison covalente entre ledit anticorps ou ledit fragment et ledit peptide, en présence d'un ou plusieurs espaceurs, étant créée entre l'azoture et l'alcyne.

Selon un mode de réalisation, dans les molécules décrites ci-dessus, plus particulièrement de la page 9, ligne 15 à la page 10, ligne 12, l'espaceur utilisé est de préférence un espaceur PEGn, n représentant de préférence un entier compris entre 1 et 10. Lorsque la molécule hybride selon l'invention comprend au moins deux espaceurs PEGn, les n des deux espaceurs peuvent être identiques ou différents. Par exemple le premier espaceur peut être un PEG₂ et le deuxième espaceur peut être un PEG₃ ou PEG₄.

Selon un mode de réalisation particulièrement préféré, dans les molécules décrites ci-dessus, plus particulièrement de la page 9, ligne 15 à la page 10, ligne 12, le peptide est dérivé de fibrine ou fibrinogène humain et l'anticorps ou le fragment d'anticorps est humain.

Selon un mode de réalisation selon l'invention, ladite molécule hybride selon l'invention est représentée par :
- un fragment d'anticorps capable de se lier à CD38 lié à un espaceur PEGn, de préférence PEG₃, lui-même couplé à un azoture lié de façon covalente à un cyclooctyne couplé à un peptide représenté par SEQ ID NO : 19 (β60-74cit_{60,72,74}) ou SEQ ID NO : 18 (α621-635cit_{621,627,630}), ou
- un fragment d'anticorps capable de se lier à CD38 lié à un espaceur PEGn, de préférence PEG₃, lui-même couplé à un azoture lié de façon covalente à un cyclooctyne couplé à un espaceur PEGn, de préférence PEG₃, lui-même lié à un peptide représenté par SEQ ID NO : 19 (β60-74cit_{60,72,74}), ou SEQ ID NO : 18 (α621-635cit_{621,627,630}),
n représentant de préférence un entier entre 1 et 10, plus particulièrement 1, 2, 3, 4 ou 8, et ledit fragment étant de préférence un fragment Fab.

Selon un mode de réalisation encore plus particulier selon l'invention, ladite molécule hybride selon l'invention est représentée par :
- un fragment d'anticorps capable de se lier à CD38 lié à un espaceur PEGn, de préférence PEG₃, lui-même couplé à un azoture lié de façon covalente à un cyclooctyne couplé à un peptide représenté par SEQ ID NO : 19 (β60-74cit_{60,72,74}), ou
- un fragment d'anticorps capable de se lier à CD38 lié à un espaceur PEGn, de préférence PEG₃, lui-même couplé à un azoture lié de façon covalente à un cyclooctyne couplé à un espaceur PEGn, de préférence PEG₃, lui-même lié à un peptide représenté par SEQ ID NO : 19 (β60-74cit_{60,72,74}), ou
- un fragment d'anticorps capable de se lier à CD38 lié à un espaceur PEGn, de préférence PEG₃, lui-même couplé à un azoture lié de façon covalente à un cyclooctyne couplé à un espaceur PEGn, de préférence PEG₃, lui-même lié à un peptide représenté par SEQ ID NO : 18 (α621-635cit_{621,627,630}),
n représentant de préférence un entier entre 1 et 10, plus particulièrement 1, 2, 3, 4 ou 8, et ledit fragment étant de préférence un fragment Fab.

Selon l'invention, la création de la liaison covalente entre l'azoture et l'alcyne correspond à une étape dite de « chimie-click », la partie N₃ de l'azoture réagissant avec un alcyne. L'azoture s'entend des sels de l'acide azothydrique HN₃, ou des azotures organiques dans lesquels un des atomes d'azote est lié de façon covalente avec un atome de carbone d'un composé organique (par exemple l'azoture de méthyle CH₃N₃). Préférentiellement l'azoture est représenté par la formule N₃. L'alcyne s'entend des molécules ayant pour formule générale CₙH₂ₙ₋₂, et qui sont caractérisées par la présence d'au moins une triple liaison. Préférentiellement l'alcyne est un cyclooctyne, encore plus préférentiellement le dibenzocyclooctyne (DBCO).

L'anticorps ou fragment, ledit peptide et éventuellement le ou les espaceur(s), sont couplés à l'alcyne ou à l'azoture par toute technique de couplage moléculaire classiquement utilisée (telle qu'une conjugaison). Toute technique peut également être utilisée pour lier de façon covalente l'anticorps ou fragment à l'espaceur et/ou le peptide à l'espaceur.

Plus particulièrement, une technique de conjugaison s'entend d'une conjugaison enzymatique ou une conjugaison chimique. Une conjugaison enzymatique s'entend par exemple d'une conjugaison à l'aide d'une transglutaminase qui catalyse la formation de liaisons covalentes entre des groupes amines libres et des résidus de glutamine ou de lysine ou encore à l'aide d'une transpeptidase telle que la sortase. Pour de plus amples informations concernant la conjugaison enzymatique, voir par exemple la demande de brevet US20160361434 ou US20170313787, ou encore la publication Ohtsuka et al., Bioscience, Biotechnology, and Biochemistry Volume 64, 2000 - Issue 12, Comparison of Substrate Specificities of Transglutaminases Using Synthetic Peptides as Acyl donors. Le substrat de la transglutaminase est par exemple un peptide comportant un résidu glutamyl (un Qtag), tel que représenté par SEQ ID NO : 29 (LLQG). L'anticorps ou le fragment peut être porteur du Qtag. Une conjugaison chimique s'entend par exemple d'une liaison covalente entre une cystéine isolée ou participant à un pont disulfure après réduction de celui-ci et par exemple un maléimide.

Selon l'invention, le terme « couplé » ou « couplage moléculaire » s'entend de l'établissement d'une liaison covalente, ainsi l'anticorps ou le fragment et/ou le peptide et/ou l'espaceur est lié de façon covalente à un alcyne ou un azoture. Le terme « lié » s'entend également d'une liaison covalente. Ainsi, à titre d'exemple, l'expression « ledit anticorps ou ledit fragment est lié à un espaceur, lui-même couplé à un azoture, et ledit peptide est également lié à un deuxième espaceur, lui-même couplé à un alcyne tel qu'un cyclooctyne», peut également se lire « ledit anticorps ou fragment est lié de façon covalente à un espaceur, lui-même lié de façon covalente à un azoture, et ledit peptide est également lié de façon covalente à un deuxième espaceur, lui-même lié de façon covalente à un alcyne tel qu'un cyclooctyne ».

### Utilisation des molécules hybrides selon l'invention

Dans un second aspect, la présente invention concerne également une molécule hybride telle que définie précédemment, pour son utilisation comme médicament.

Plus particulièrement, selon l'invention, lesdites molécules hybrides sont destinées à cibler et lyser dans l'organisme des patients, par apoptose, par phagocytose, par ADCC ou par activation du complément, les plasmocytes sécréteurs d'ACPA. En effet, la molécule hybride selon l'invention se lie aux plasmocytes grâce à l'anticorps ou fragment qui se lie à CD38 et/ou CD138. La molécule hybride selon l'invention se lie également aux ACPA grâce au peptide dérivé de fibrine ayant un résidu citrullyl : il s'agit de la cible desdits ACPA. Les ACPA vont permettre le pontage des molécules hybrides selon l'invention fixées à la surface des plasmocytes et ainsi induire leur apoptose. Les ACPA, comme tout anticorps, possèdent des fonctions effectrices qui s'activent après liaison à leur antigène-cible, participant ainsi à la destruction plasmocytaire par phagocytose et/ou par ADCC et/ou par activation du complément.

Selon un mode de réalisation particulier, l'invention concerne une molécule hybride, telle que définie précédemment pour son utilisation dans le traitement des maladies auto-immunes associées à la production d'auto-anticorps anti-protéines citrullinées, en particulier le syndrome de Gougerot-Sjögren et la polyarthrite rhumatoïde. Dans ce mode de réalisation, les formes sévères desdites maladies auto-immunes associées à la production d'auto-anticorps anti-protéines citrullinées sont ciblées, de même que les formes dites « frontières » avec d'autres arthrites chroniques, telles que l'arthrite psoriasique ou le lupus érythémateux disséminé.

Selon un mode de réalisation, l'invention concerne également une composition pharmaceutique comprenant une molécule hybride selon l'une quelconque des revendications précédentes, en combinaison avec un véhicule pharmaceutiquement acceptable.

Selon l'invention « un véhicule pharmaceutiquement acceptable » s'entend de toute formulation rendant la composition apte à être administrée à un patient, sous n'importe quelle forme galénique.

Comme indiqué ci-dessus, la présente invention vise à cibler les plasmocytes sécréteurs d'ACPA. Cependant, les lymphocytes B ACPA-positifs précurseurs des plasmocytes, avant leur différenciation, ne sont pas ou peu ciblés par lesdites molécules hybrides selon l'invention. Selon un mode de réalisation particulier, la présente invention concerne ainsi une molécule hybride telle que précédemment décrite, pour son utilisation comme médicament, en combinaison avec une deuxième molécule hybride, ladite deuxième molécule hybride comprenant au moins un fragment Fc d'anticorps lié de façon covalente à au moins un peptide dérivé de fibrine porteur d'au moins un résidu citrullyl, un espaceur étant optionnellement présent entre ledit fragment Fc et ledit peptide. Une telle thérapie de combinaison permet de cibler et détruire spécifiquement les clones plasmocytaires sécréteurs d'ACPA et les lymphocytes B ACPA-positifs qui sont leurs précurseurs, et ainsi de faire disparaître les ACPA de l'organisme des patients et d'empêcher leur réapparition.

Selon un mode de réalisation particulier, dans ladite deuxième molécule hybride selon l'invention, ledit fragment Fc est un fragment Fc humain, notamment d'IgG, plus particulièrement d'IgG1. L'IgG1 peut appartenir à tous les allotypes, par exemple G1m3 ou nG1m1. A titre d'exemple, le fragment Fc d'IgG1 est représenté par SEQ ID NO : 24, SEQ ID NO : 25 (Fc+Qtag) ou SEQ ID NO : 26 (Fc+Qtag bis).

Selon un mode de réalisation, dans ladite deuxième molécule hybride selon l'invention, ledit fragment Fc est de type sauvage ou muté. La ou les mutations peuvent viser à augmenter la demi-vie plasmatique, la diminuer, modifier les fonctions effectrices du fragment Fc, ...

Selon un mode de réalisation encore plus particulier, ledit fragment Fc muté comprend au moins les mutations suivantes :
- L234A et L235A (LALA), ou
- L234A, L235A et P329G (LALAPG), ou
- G236A, S239D et I332E (GASDIE), ou
- G236A, S239D, A330L et I332E (GASDALIE), ou
- S239D, H268F, S324T et I332E (SDHFSTIE ou SDH),
la numérotation étant indiquée dans la séquence d'une IgG1 humaine selon l'index EU. De telles mutations sont notamment décrites dans l'article Bruhns and Jönsson, Immunol Rev. 2015 Nov;268(1):25-51. De préférence, lorsque la molécule hybride est utilisée en thérapie, ledit fragment Fc muté comprend au moins les mutations GASDIE, GASDALIE, ou SDH.

Selon un mode de réalisation particulier, dans ladite deuxième molécule hybride selon l'invention, ledit fragment Fc présente un taux de fucosylation compris entre 0% à 100% des formes glycosylées. Selon l'invention « entre 0% et 100% » représente toutes les valeurs entières comprises entre 0 et 100, i.e. ; 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 et 100%. Une faible fucosylation du fragment Fc provoque une forte réponse ADCC. C'est pourquoi selon un mode de réalisation particulier, ledit fragment Fc présente un taux de fucosylation compris entre 0% à 60% des formes glycosylées, notamment 50%, 40%, 30%, 20%, 10% ou 0%. Selon l'invention, le taux de fucosylation est défini comme la proportion moyenne de fucose portée par le fragment Fc, par rapport à la quantité maximale de fucose que peut porter un fragment Fc.

Selon ce mode de réalisation particulier de l'invention, la première et la deuxième molécule hybride sont administrées de façon simultanée, séparée ou étalée dans le temps.

Selon un autre mode de réalisation, la molécule hybride selon l'invention peut être couplée à au moins un radioisotope ou à au moins un fluorochrome, tels que A488 ou A647. De telles molécules peuvent avantageusement être utilisées comme outils moléculaires traceurs.

Selon un autre mode de réalisation, l'invention concerne ainsi l'utilisation *in vitro* ou ex *vivo* d'une molécule hybride comprenant au moins un peptide dérivé de fibrine ayant au moins un résidu citrullyl, ledit peptide étant lié de façon covalente à au moins un anticorps ou à au moins un fragment d'anticorps, ledit anticorps ou fragment étant capable de se lier à CD38 et/ou CD138, un ou plusieurs espaceurs étant optionnellement présents entre ledit peptide et ledit anticorps ou ledit fragment, comme outil moléculaire. De telles constructions peuvent notamment être utilisées pour analyser la fixation des molécules hybrides aux ACPA et aux plasmocytes, ainsi que pour analyser la réactivité des plasmocytes à la fixation des hybrides suivie du pontage aux ACPA.... Les radioisotopes et/ou fluorochromes sont de préférence couplés sur le fragment d'anticorps.

### Procédé de production des molécules hybrides selon l'invention

Dans un autre aspect, l'invention concerne également un procédé permettant d'obtenir une molécule hybride telle que définie précédemment.

Selon un mode de réalisation, l'invention concerne ainsi un procédé de production d'une molécule hybride telle que définie précédemment, comprenant les étapes suivantes :
- (i) obtention d'un azoture couplé à un anticorps ou à un fragment capable de se lier à CD38 et/ou CD138, ou obtention d'un alcyne couplé à un anticorps ou à un fragment capable de se lier à CD38 et/ou CD138,
- (ii) obtention d'un alcyne couplé à un peptide ou obtention d'un azoture couplé à un peptide,
- (iii) mélange des produits générés en (i) et (ii), la liaison covalente s'établissant spécifiquement entre l'azoture et l'alcyne,
- l'étape (i) pouvant être réalisée avant ou après l'étape (ii), ou bien de façon concomitante.

Selon un mode de réalisation, l'invention concerne ainsi un procédé de production d'une molécule hybride telle que définie précédemment, comprenant les étapes suivantes :
- (i) couplage d'un azoture à 'un anticorps ou à un fragment capable de se lier à CD38 et/ou CD138, éventuellement en présence d'un espaceur,
- (ii) couplage d'un alcyne à un peptide, éventuellement en présence d'un espaceur,
- (iii) mélange des produits générés en (i) et (ii), la liaison covalente s'établissant spécifiquement entre l'azoture et l'alcyne.
- l'étape (i) pouvant être réalisée avant ou après l'étape (ii), ou bien de façon concomitante.

Selon un autre mode de réalisation, l'invention concerne aussi un procédé de production d'une molécule hybride telle que définie précédemment, comprenant les étapes suivantes :
- (i) couplage d'un alcyne à un anticorps ou à un fragment capable de se lier à CD38 et/ou CD138, éventuellement en présence d'un espaceur,
- (ii) couplage d'un azoture à un peptide, éventuellement en présence d'un espaceur,
- (iii) mélange des produits générés en (i) et (ii), la liaison covalente s'établissant spécifiquement entre l'azoture et l'alcyne, l'étape (i) pouvant être réalisée avant ou après l'étape (ii), ou bien de façon concomitante.

Selon un autre mode de réalisation, l'invention concerne aussi un procédé de production d'une molécule hybride telle que définie précédemment, comprenant les étapes suivantes :
- (i) couplage d'au moins un alcyne ou un azoture sur chaque monomère de l'anticorps ou du fragment capable de se lier à CD38 et/ou CD138, éventuellement en présence d'un espaceur,
- (ii) couplage d'au moins un azoture ou un alcyne au peptide, éventuellement en présence d'un espaceur,
- (iii) mélange des produits générés en (i) et (ii), la liaison covalente s'établissant spécifiquement entre l'azoture et l'alcyne, l'étape (i) pouvant être réalisée avant ou après l'étape (ii), ou bien de façon concomitante.

Les séquences de l'invention sont représentées dans le Tableau 1 ci-après.

**[Tableau 1]. Tableau récapitulatif des séquences de l'invention**

| Numéro de la séquence / Nom de la séquence le cas échéant | Séquence |
|---|---|
| SEQ ID NO: 1 | X₁PAPPPISGGGYX₂AX₃ |
| SEQ ID NO : 2 | GPX₁VVEX₂HQSACKDS |
| SEQ ID NO : 3 | SGIGTLDGFX₁HX₂HPD |
| SEQ ID NO : 4 | VDIDIKIX₁SCX₂GSCS |
| SEQ ID NO : 12 | X₁GHAKSX₂PVX₃GIHTS |
| SEQ ID NO : 5 / α36-50cit_{38,42} | GPXVVEXHQSACKDS, X étant un résidu citrullyl |
| SEQ ID NO : 6 / α171-185cit_{178,181} | VDIDIKIXSCXGSCS, X étant un résidu citrullyl |
| SEQ ID NO : 7 / α183-197cit_{186,190} | SCSXALAXEVDLKDY, X étant un résidu citrullyl |
| SEQ ID NO : 7 / α246-260cit₂₅₈ | PEWKALTDMPQMXME, X étant un résidu citrullyl |
| SEQ ID NO : 9 / α259-273cit_{263,271} | MELEXPGGNEITXGG, X étant un résidu citrullyl |
| SEQ ID NO : 10 / α366-380cit₃₆₇ | EXGSAGHWTSESSVS, X étant un résidu citrullyl |
| SEQ ID NO : 11 / α396-410cit₄₀₄ | DSPGSGNAXPNNPDW, X étant un résidu citrullyl |
| SEQ ID NO : 13 / α411-425cit₄₂₅ | GTFEEVSGNVSPGTX, X étant un résidu citrullyl |
| SEQ ID NO : 14 / α501-515cit_{510,512} | SGIGTLDGFXHXHPD, X étant un résidu citrullyl |
| SEQ ID NO : 15 / α546-560cit₅₄₇ | SXGSESGIFTNTKES, X étant un résidu citrullyl |
| SEQ ID NO : 16 / α561-575cit₅₇₃ | SSHHPGIAEFPSXGK, X étant un résidu citrullyl |
| SEQ ID NO : 17 / α588-602cit₅₉₁ | SYNXGDSTFESKSYK, X étant un résidu citrullyl |
| SEQ ID NO : 18 / α621-635cit_{621,627,630} | XGHAKSXPVXGIHTS, X étant un résidu citrullyl |
| SEQ ID NO : 19 / β60-74cit_{60,72,74} | XPAPPPISGGGYXAX, X étant un résidu citrullyl |
| SEQ ID NO : 20 / β210-224cit₂₂₄ | QKLESDVSAQMEYCX, X étant un résidu citrullyl |
| SEQ ID NO : 21 / β281-295cit_{285,294} | VIQNXQDGSVDFGXK, X étant un résidu citrullyl |
| SEQ ID NO : 22 / β420-434cit₄₂₁ | PXKQCSKEDGGGWWY, X étant un résidu citrullyl |
| SEQ ID NO : 23 / β433-447cit_{436,445} | WYNXCHAANPNGXYY, X étant un résidu citrullyl |
| SEQ ID NO : 27 / Chaine α de fibrine | GPRVVERHQSACKDSDWPFCSDEDWNYKCPSGCRMKGLIDEVNQDFTNRINKLKNSL FEYQKNNKDSHSLTTNIMEILRGDFSSANNRDNTYNRVSEDLRSRIEVLKRKVIEKVQHI QLLQKNVRAQLVDMKRLEVDIDIKIRSCRGSCSRALAREVDLKDYEDQQKQLEQVIAKDL LPSRDRQHLPLIKMKPVPDLVPGNFKSQLQKVPPEWKALTDMPQMRMELERPGGNEIT RGGSTSYGTGSETESPRNPSSAGSWNSGSSGPGSTGNRNPGSSGTGGTATWKPGSS GPGSTGSWNSGSSGTGSTGNQNPGSPRPGSTGTWNPGSSERGSAGHWTSESSVSG STGQWHSESGSFRPDSPGSGNARPNNPDWGTFEEVSGNVSPGTRREYHTEKLVTSK GDKELRTGKEKVTSGSTTTTRRSCSKTVTKTVIGPDGHKEVTKEVVTSEDGSDCPEAM DLGTLSGIGTLDGFRHRHPDEAAFFDTASTGKTFPGFFSPMLGEFVSETESRGSESGIF TNTKESSSHHPGIAEFPSRGKSSSYSKQFTSSTSYNRGDSTFESKSYKMADEAGSEAD HEGTHSTKRGHAKSRPVRGIHTS |
| SEQ ID NO : 28 / Chaine β de fibrine | GHRPLDKKREEAPSLRPAPPPISGGGYRARPAKAAATQKKVERKAPDAGGCLHADPDL GVLCPTGCQLQEALLQQERPIRNSVDELNNNVEAVSQTSSSSFQYMYLLKDLWQKRQK QVKDNENVVNEYSSELEKHQLYIDETVNSNIPTNLRVLRSILENLRSKIQKLESDVSAQM EYCRTPCTVSCNIPVVSGKECEEIIRKGGETSEMYLIQPDSSVKPYRVYCDMNTENGGW TVIQNRQDGSVDFGRKWDPYKQGFGNVATNTDGKNYCGLPGEYWLGNDKISQLTRMG PTELLIEMEDWKGDKVKAHYGGFTVQNEANKYQISVNKYRGTAGNALMDGASQLMGE NRTMTIHNGMFFSTYDRDNDGWLTSDPRKQCSKEDGGGWWYNRCHAANPNGRYYW GGQYTWDMAKHGTDDGVVWMNWKGSWYSMRKMSMKIRPFFPQQ |
| SEQ ID NO : 24 / Fragment Fc | APELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQV YTLPPSREEMTKNQVSLTCLVKGFYPSDIAVEWESNGQPENNYKTTPPVLDSDGSFFLY SKLTVDKSRWQQGNVFSCSVMHEALHNHYTQKSLSLSPGK |
| SEQ ID NO : 25 / Fragment Fc + Qtag | LLQGARSDATHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKALP APIEKTISKAKGQPREPQVYTLPPSREEMTKNQVSLTCLVKGFYPSDIAVEWESNGQPE NNYKTTPPVLDSDGSFFLYSKLTVDKSRWQQGNVFSCSVMHEALHNHYTQKSLSLSPG K |
| SEQ ID NO : 26 / Fragment Fc + Qtag bis | AHGHGHGLLQGARSDATHTCPPCPAPELLGGPSVFLFPPKPKDTLMISRTPEVTCVVVD VSHEDPEVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDIAVEWE SNGQPENNYKTTPPVLDSDGSFFLYSKLTVDKSRWQQGNVFSCSVMHEALHNHYTQK SLSLSPGK |
| SEQ ID NO : 29 (Exemple de Qtag) | LLQG |
| SEQ ID NO : 30 Chaine α de fibrinogène | MFSMRIVCLVLSVVGTAWTADSGEGDFLAEGGGVRGPRVVERHQSACKDSDWPFCSD EDWNYKCPSGCRMKGLIDEVNQDFTNRINKLKNSLFEYQKNNKDSHSLTTNIMEILRGD FSSANNRDNTYNRVSEDLRSRIEVLKRKVIEKVQHIQLLQKNVRAQLVDMKRLEVDIDIKI RSCRGSCSRALAREVDLKDYEDQQKQLEQVIAKDLLPSRDRQHLPLIKMKPVPDLVPGN FKSQLQKVPPEWKALTDMPQMRMELERPGGNEITRGGSTSYGTGSETESPRNPSSAG SWNSGSSGPGSTGNRNPGSSGTGGTATWKPGSSGPGSTGSWNSGSSGTGSTGNQN PGSPRPGSTGTWNPGSSERGSAGHWTSESSVSGSTGQWHSESGSFRPDSPGSGNA RPNNPDWGTFEEVSGNVSPGTRREYHTEKLVTSKGDKELRTGKEKVTSGSTTTTRRSC SKTVTKTVIGPDGHKEVTKEVVTSEDGSDCPEAMDLGTLSGIGTLDGFRHRHPDEAAFF DTASTGKTFPGFFSPMLGEFVSETESRGSESGIFTNTKESSSHHPGIAEFPSRGKSSSY SKQFTSSTSYNRGDSTFESKSYKMADEAGSEADHEGTHSTKRGHAKSRPVRGIHTS |
| SEQ ID NO : 31 Chaine β de fibrinogène | |

D'autres caractéristiques, détails et avantages de l'invention apparaîtront à la lecture des Figures annexées et des exemples qui illustrent l'invention et ne visent en aucun cas à la limiter.

Dans les molécules hybrides exemplifiées, c'est l'extrémité Nterminale du fragment Fab qui est liée à un espaceur PEGn, et c'est toujours l'extrémité Nterminale dudit peptide qui est lié au cycloctyne DBCO ou à un deuxième espaceur PEGn le cas échéant. De plus, dans les molécules hybrides exemplifiées, dans l'espaceur PEGn lié au fragment Fab, n représente plus particulièrement 3 ou 4, et dans l'espaceur PEGn lié au peptide, l'espaceur PEGn représente 3 ou 8, bien que toute autre valeur de n, notamment entre 1 et 10, puisse être utilisée.

### Brève description des Figures

**Fig. 1**

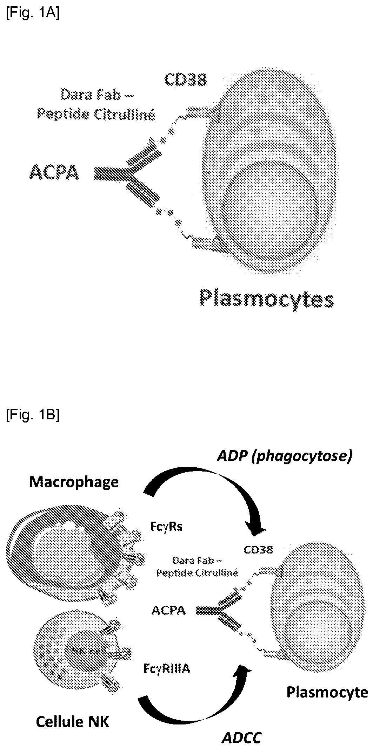

   [Fig. 1A] **représente un plasmocyte qui exprime à sa surface les molécules CD38 sur lesquelles sont liées une molécule hybride Dara-Fab-peptide citrulliné selon l'invention (un fragment Fab de l'anticorps Daratumumab lié de façon covalente à un peptide citrulliné).**
   Ladite molécule hybride est elle-même liée par le peptide citrulliné à un ACPA. Le pontage des molécules hybrides Dara-Fab-peptide citrulliné par les ACPA est cytotoxique et entraine la mort du plasmocyte.
   [Fig. 1B] **représente la phagocytose par des macrophages (ADP) ou cytotoxicité induite par des cellules NK (ADCC), d'un plasmocyte CD38+ après pontage par les ACPA de molécules hybrides Dara-Fab-peptide citrulliné à la surface d'un plasmocyte.**
   Un plasmocyte exprime à sa surface les molécules CD38 sur lesquelles sont liées une molécule hybride Dara-Fab-peptide citrulliné selon l'invention, ladite molécule hybride étant elle-même liée par le peptide citrulliné à un ACPA. L'engagement du fragment Fc des ACPA à différents FcyRs exprimés à la surface d'un macrophage ou aux FcγRIIIA exprimés à la surface des cellules NK, va activer la phagocytose (ADP) et/ou la cytotoxicité induite par les anticorps (ADCC), conduisant à la destruction spécifique du plasmocyte.
**Fig. 2**
   [Fig. 2] **représente le schéma d'un exemple de molécule hybride selon l'invention.**
   Un espaceur (qui est optionnel) est représenté entre l'anticorps ou fragment d'anticorps qui se lie à CD38 (Dara) et le peptide dérivé de fibrine ayant un résidu citrullyl (dénommé « peptide citrulliné »). Une construction avec un fragment F(ab)'2 est également représentée. A titre d'exemple le F(ab)'2 peut se lier à la fois à CD38 et à CD138. Fab : Fragment Antigen Binding. Dara Fab : Fab d'un anticorps spécifique de la molécule CD38 (Daratumumab).
**Fig. 3**
   [Fig. 3] **représente la réactivité de plusieurs molécules hybrides comprenant le peptide de SEQ ID NO** : **19 (β60-74cit_{60,72,74}) vis-à-vis des ACPA anti-β60-74 purifiés.**
   β60-74 représente le peptide de SEQ ID NO : 19. DaraFab-β60-74 représente un Fab capable de se lier à CD38 lié à un espaceur PEGn, lui-même couplé à un azoture qui est lié de façon covalente à un cyclooctyne (DBCO) qui est couplé à un peptide de SEQ ID NO : 19. DaraFab-PEG-β60-74 représente un Fab capable de se lier à CD38 lié à un espaceur PEGn, lui-même couplé à un azoture qui est lié de façon covalente à un cyclooctyne (DBCO) qui est couplé à un espaceur PEGn qui est lui-même lié à un peptide de SEQ ID NO : 19. n représente un nombre entre 1 et 10 lorsqu'un espaceur PEGn est utilisé, de préférence 1, 2, 3, 4 ou 8.
**Fig. 4**

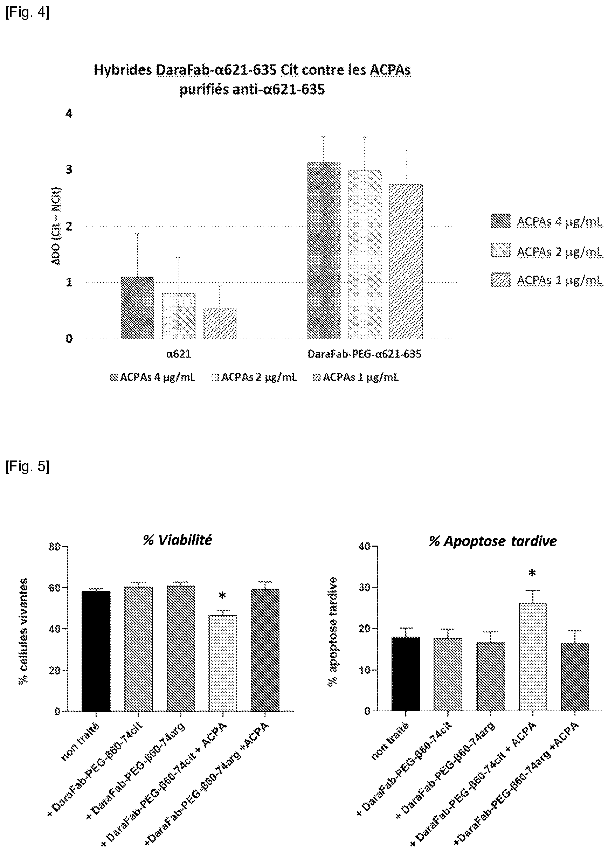

   [Fig. 4] **représente la réactivité de plusieurs molécules hybrides comprenant le peptide de SEQ ID NO : 18 (α621) vis-à-vis d'ACPA anti-α621-635 purifiés.**
   α621 représente le peptide de SEQ ID NO : 18. DaraFab-PEG-α621-635 représente un Fab capable de se lier à CD38 lié à un espaceur PEGn, lui-même couplé à un azoture qui est lié de façon covalente à un cyclooctyne (DBCO) qui est couplé à un espaceur PEGn qui est lui-même lié à un peptide de SEQ ID NO : 18. n représente un nombre entre 1 et 10, de préférence 1, 2, 3, 4 ou 8.
**Fig. 5**
   [Fig. 5] **représente la cytotoxicité du DaraFab-PEG-β60-74Cit vis-à-vis de la lignée B BAEV (BC-9) produite à partir de lymphocytes d'un patient atteint de polyarthrite rhumatoïde, après pontage par des ACPAs anti-β60-74 purifiés.**
   La partie gauche représente le pourcentage de cellules vivantes et la partie droite, le pourcentage de cellules en apoptose tardive.
**Fig. 6**

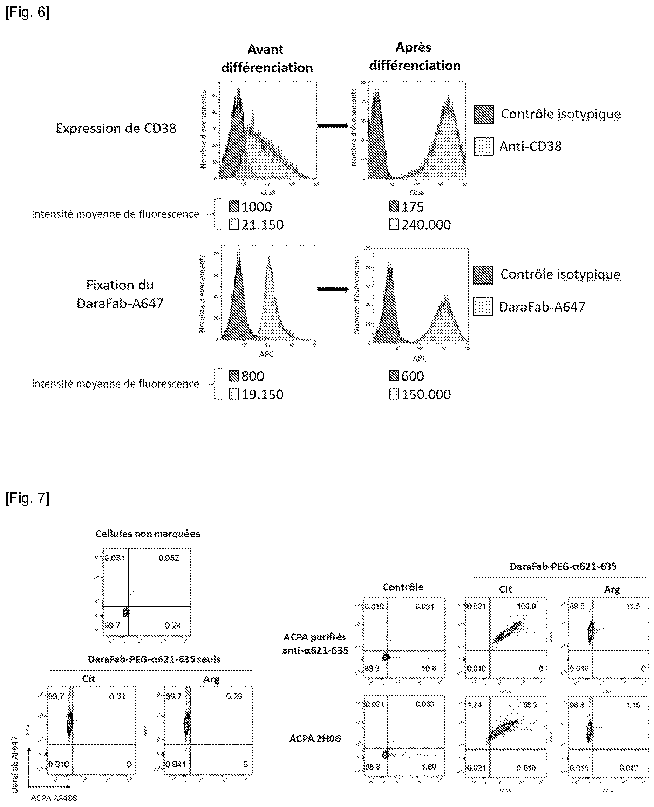

   [Fig. 6] **représente l'expression de CD38 et la liaison du Fab Dara** (Fragment Fab qui se lie à CD38 marqué avec le fluorochrome A647) **sur les cellules BC-9 avant (à gauche) et après (à droite) différenciation.**
**Fig. 7**
   [Fig. 7] **représente l'interaction des DaraFab-PEG-α621-635 et des ACPA purifiés anti-α621-635cit ou de l'ACPA 2H06** à **la surface de la lignée myélomateuse KMS-12-PE.**
   DaraFab-PEG-α621-635 représente un Fab capable de se lier à CD38 lié à un espaceur PEGn, lui-même couplé à un azoture qui est lié de façon covalente à un cyclooctyne (DBCO) qui est couplé à un espaceur PEGn qui est lui-même lié à un peptide de SEQ ID NO : 18. n représente un nombre entre 1 et 10, de préférence 1, 2, 3, 4 ou 8.
**Fig. 8**

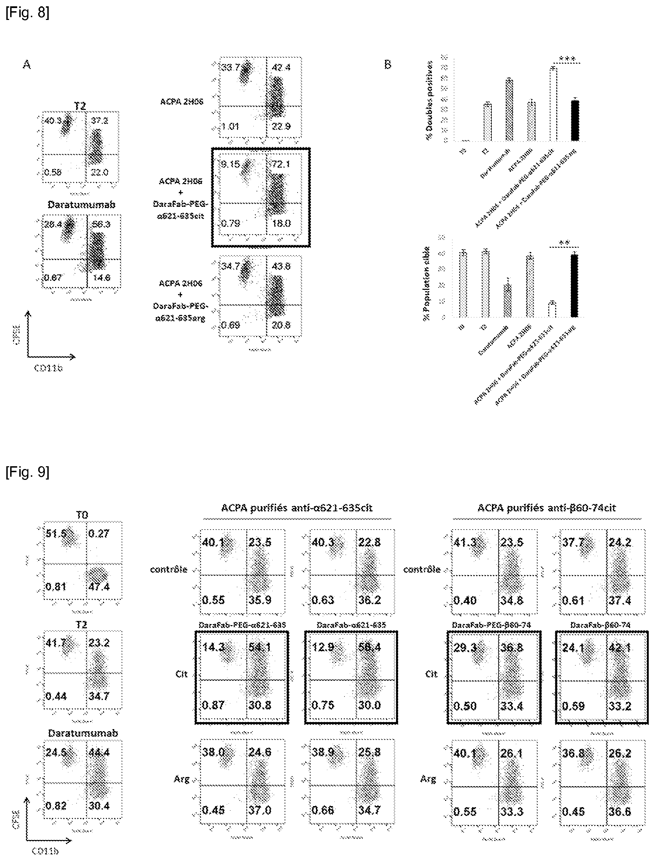

   [Fig. 8] **représente la phagocytose de la lignée myélomateuse différenciée KMS-12-PE par des macrophages en présence des hybrides DaraFab-PEG-α621-635 Cit et de l'ACPA 2H06.**
   DaraFab-PEG-α621-635cit représente la molécule hybride telle que décrite en Figure 7. DaraFab-PEG-α621-635arg représente un Fab capable de se lier à CD38 lié à un espaceur PEGn, lui-même couplé à un azoture qui est lié de façon covalente à un cyclooctyne (DBCO) qui est couplé à un espaceur PEGn qui est lui-même lié à un peptide de SEQ ID NO : 18 dans lequel X représente des résidus arginyls (i.e. le peptide n'est pas citrulliné).
**Fig. 9**
   [Fig. 9] **représente la phagocytose de la lignée myélomateuse différenciée KMS-12-PE par des macrophages en présence de DaraFab-PEG-β60-74 ou de DaraFab-PEG-α621-635 et, respectivement, des ACPA purifiés anti-β60-74cit ou anti-α621-635cit.**
   DaraFab-PEG-α621-635 est la molécule telle que décrite en Figure 7. DaraFab-α621-635 représente un Fab capable de se lier à CD38 lié à un espaceur PEGn, lui-même couplé à un azoture qui est lié de façon covalente à un cyclooctyne (DBCO) qui est couplé à un peptide de SEQ ID NO : 18. DaraFab-PEG-β60-74 représente un Fab capable de se lier à CD38 lié à un espaceur PEGn, lui-même couplé à un azoture qui est lié de façon covalente à un cyclooctyne (DBCO) qui est couplé à un espaceur PEGn qui est lui-même lié à un peptide de SEQ ID NO : 19 (n représente un nombre entre 1 et 10, de préférence 1, 2, 3, 4 ou 8). DaraFab-β60-74 représente un Fab capable de se lier à CD38 lié à un espaceur PEGn, lui-même couplé à un azoture qui est lié de façon covalente à un cyclooctyne (DBCO) qui est couplé à un peptide de SEQ ID NO : 19.

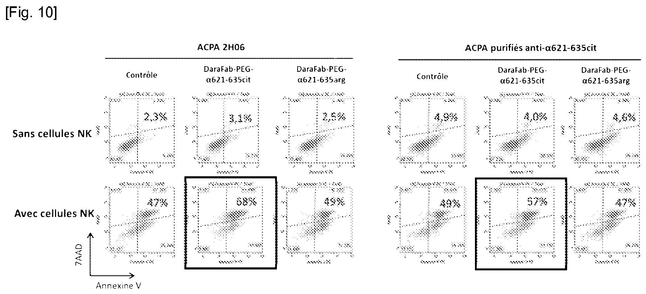

**Fig. 10**Fig. 10] **représente l'induction d'apoptose de la lignée myélomateuse différenciée KMS-12-PE par des cellules NK en présence de DaraFab-PEG-α621-635 et d'ACPA purifiés anti-α621-635cit ou d'ACPA 2H06.**

Les molécules hybrides sont celles décrites en Figure 8.

### Exemples

### Exemple 1 : Exemple de production d'une molécule hybride selon l'invention

La molécule hybride ici décrite comprend la construction suivante : un fragment Fab lié de façon covalente à un PEGn, lui-même couplé à un azoture, ledit azoture étant lié de façon covalente à un alcyne, qui est lui-même lié à un espaceur couplé à un peptide citrulliné. Une telle molécule peut se lire : Fab-PEGn-N₃-DBCO-PEGn-peptide citrulliné.
1. Synthèse d'un NH2-PEGn-N₃ (i.e. un espaceur couplé à un azoture à une extrémité et possédant une fonction amine libre à une autre extrémité).
2. Mise en présence du NH₂-PEGn-N₃, avec un fragment Fab possédant un Qtag, et une transglutaminase, de préférence pendant 16h à 37°C. Cette étape dite « de dérivation » est de préférence réalisée avec 10 fois plus de moles de NH₂-PEGn-N₃ que de moles de fragment Fab. La transglutaminase est utilisée, à hauteur de 15U/µmol par Qtag présent. Eventuellement un dessalage peut être réalisé pour retirer l'excédent d'espaceur non lié au fragment Fab à l'issue de l'étape. Un Fab-PEGn-N₃ est ainsi obtenu. Si l'anticorps ou le fragment comporte 2Qtag (un porté sur chaque monomère), alors l'anticorps ou le framgent peut porter deux PEGn-N₃
3. Synthèse d'un Cys-PEGn-peptide citrulliné (i.e. un espaceur couplé à un peptide à une extrémité et possédant une cystéine libre à une autre extrémité). Le DBCO est ensuite couplé au Cys-PEGn-peptide citrulliné au niveau de la cystéine, et un DBCO-PEGn-peptide citrulliné est ainsi obtenu.
4. Réalisation du « click » : couplage entre le N₃ et le DBCO. Le DBCO-PEGn-peptide citrulliné est mis en présence du Fab-PEGn-N₃, en utilisant de préférence 10 fois plus de moles de DBCO-PEGn-peptide citrulliné que de moles de Fab-PEGn-N₃. La réaction du click se déroule à température ambiante et est presque complète au bout de 4h.
5. Obtention de la molécule hybride : Fab-PEGn-N₃-DBCO-PEGn-peptide citrulliné.

De façon similaire un Fab-PEGn-DBCO et un N₃-PEGn-peptide citrulliné peuvent être obtenus, puis couplés pour obtenir Fab-PEGn-DBCO-N₃-PEGn-peptide citrulliné.

De façon similaire un Fab-PEGn-DBCO et un N₃-peptide citrulliné peuvent être obtenus, ou un Fab-PEGn-N₃ et un DBCO-peptide citrulliné, puis couplés pour obtenir respectivement Fab-PEGn-DBCO-N₃-peptide citrulliné ou Fab-PEGn-N₃-DBCO-peptide citrulliné.

Le même procédé peut être utilisé avec un anticorps ou un autre type de fragment d'anticorps.

### Exemple 2 : Réactivité des ACPA purifiés sur peptides citrullinés, vis-à-vis des hybrides DaraFab-peptide Cit

Les ACPA (ici ACPA, fraction pH3) utilisés sont des ACPA obtenus à partir de sérum de patients atteints de polyarthrite rhumatoïde ACPA-positifs. De tels ACPA ont été obtenus par une méthode classique de chromatographie d'affinité sur colonne, connue de l'homme de l'art, utilisant comme antigènes liés, un ou plusieurs des cinq peptides immunodominants de l'invention (cf SEQ ID NO : 5, SEQ ID NO : 6, SEQ ID NO : 14, SEQ ID NO : 18 ou SEQ ID NO : 19).

### a. Exemples avec des molécules hybrides contenant le peptide β60-74cit

Des plaques de microtitration ELISA ont été revêtues à l'aide de 100 µL/puits d'une solution contenant soit le peptide β60-74cit (SEQ ID NO : 19), soit une molécule hybride : DaraFab-β60-74 ou DaraFab-PEG-β60-74. Ces solutions ont été utilisées chacune à la concentration de 5 µg/mL en tampon PBS (Phosphate Buffered Saline) pendant une nuit à 4°C. Le peptide non citrulliné et les molécules hybrides construites avec le peptide non citrulliné (β60-74arg) ont été utilisés à la même concentration, comme contrôles. Une saturation des puits a ensuite été réalisée en tampon PBS 2% BSA (Bovine Serum Albumin), pendant 1h à 4°C. Après lavages, les ACPA purifiés ont été incubés à 1, 0,5 ou 0,25 µg/mL dilués en tampon PBS 2% BSA 2M NaCl pendant 1h à 4°C. Après lavages, la réactivité des ACPA a été détectée à l'aide d'un anticorps secondaire F(ab')2 anti-Fc d'IgG humaines dilué au 1/10.000 en tampon PBS 2% BSA. Les résultats sont exprimés en ΔDO (Delta-Densité Optique), correspondant à la DO obtenue avec le peptide β60-74cit ou avec les molécules hybrides construites avec le peptide β60-74cit, soustraite respectivement de la DO obtenue avec le peptide β60-74arg ou avec les molécules hybrides construites avec le peptide β60-74arg.

Les résultats sont présentés en Figure 3. Les résultats montrent une réactivité dose-dépendante des ACPA purifiés sur peptide β60-74 vis-à-vis des molécules hybrides DaraFab-β60-74 et DaraFab-PEG-β60-74. La réactivité des ACPA vis-à-vis du peptide β60-74 est comparativement plus faible, alors que la densité épitopique qu'il présente est supérieure à celles des molécules hybrides. L'accessibilité des épitopes est donc meilleure sur les molécules hybrides.

### b. Exemples avec des molécules hybrides contenant le peptide α621-635cit

Des plaques ELISA ont été revêtues de 100 µL/puits d'une solution contenant soit le peptide α621-635cit (SEQ ID NO : 18), soit une molécule hybride DaraFab-PEG-α621-635, utilisées à la concentration de 5 µg/mL en tampon PBS (Phosphate Buffered Saline) pendant une nuit à 4°C. Le peptide non citrulliné α621-635arg et les molécules hybrides construites avec le peptide non citrulliné (α621-635arg), ont été utilisés comme contrôles à la même concentration. Une saturation des puits a ensuite été réalisée en tampon PBS 2% BSA (Bovine Serum Albumin), pendant 1h à 4°C. Après lavages, les ACPA purifiés ont été incubés à 4, 2 et 1 µg/mL dilués en tampon PBS 2% BSA 2M NaCl pendant 1h à 4°C. Après lavages, la réactivité des ACPA a été détectée à l'aide d'un anticorps secondaire F(ab')2 anti-Fc d'IgG humaines dilué au 1/10.000 en tampon PBS 2% BSA. Les résultats sont exprimés en ΔDO (Delta-Densité Optique), correspondant à la DO obtenue avec le peptide α621-635cit ou avec les molécules hybrides construites avec le peptide α621-635cit, soustraite respectivement de la DO obtenue avec le peptide α621-635arg ou avec les molécules hybrides construites avec le peptide α621-635arg.

Les résultats sont présentés en Figure 4. Les résultats montrent une forte réactivité dose-dépendante des ACPA purifiés sur peptide α621-635Cit vis-à-vis des molécules hybrides DaraFab-PEG-α621-635cit. La réactivité des ACPA vis-à-vis du peptide α621-635cit est comparativement plus faible, alors que sa densité épitopique est supérieure à celle des molécules hybrides. L'accessibilité des épitopes est donc meilleure sur les molécules hybrides.

### Exemple 3 : Cytotoxicité du Dara-Fab peptide β60Cit vis-à-vis de cellules de la lignée Daudi (CD38+), induite par les ACPAs purifiés sur β60-74 élués à pH3 (pontage des hybrides)

Les ACPA sont obtenus comme dans l'Exemple 2.

DaraFab-peptideCIT ici représente un Fab capable de se lier à CD38 lié à un espaceur PEGn, lui-même couplé à un azoture qui est lié de façon covalente à un cyclooctyne (DBCO) qui est couplé à un peptide de SEQ ID NO : 19. DaraFab-peptideNCIT ici représente un Fab capable de se lier à CD38 lié à un espaceur PEGn, lui-même couplé à un azoture qui est lié de façon covalente à un cyclooctyne (DBCO) qui est couplé à un peptide de SEQ ID NO : 19 dans laquelle Iles résidus arginyl n'ont pas été substitués par des résidus citrullyl (i.e. le peptide n'est pas citrulliné). n représente un nombre entre 1 et 10, de préférence 1, 2, 3, 4 ou 8.

Des cellules issues de la lignée Daudi (CD38+) ont été déposées en plaques de 96 puits, à raison de 50.000 cellules/puits en milieu RPMI complet (10% SVF, 1% glutamine, 1% pénicilline/streptomycine) et incubées durant 1h à 37°C avec 10µg/mL des hybrides DaraFab-peptideCIT ou DaraFab-peptideNCIT. Après lavage, ont été ajoutées des IgG humaines (contrôle négatif) ou des ACPA anti-β60-74 purifiés élués à pH3, à 5 µg/mL durant 20h à 37°C, avant de procéder à un marquage par l'Annexine-V (marqueur d'apoptose). Les cellules ont ensuite été prélevées et analysées par cytométrie en flux (BD Biosciences, CANTO II). Les valeurs montrées dans les 3 premières colonnes correspondent au pourcentage de cellules Annexine-V-positives. Δ(CIT-NCIT) (%) représente la différence entre les pourcentages de cellules Annexine-V-positives en présence de DaraFab-peptideCIT versus NCIT,divisée par le pourcentage de cellules Annexine-V-positives en présence de DaraFab-β60-74peptideNCIT. Cet indicateur montre la modulation de l'apoptose en présence d'IgG ou d'ACPA.

Les résultats sont présentés dans le Tableau 2.

**[Tableau 2]. Tableau 2 représente la quantification de l'apoptose de cellules de la lignée Daudi (CD38+) revêtues du DaraFab-β60-74CIT, après pontage par les ACPAs purifiés élués à pH3. Synthèse de 4 expériences indépendantes.**

| Anticorps | Non traité | DaraFab-peptideCIT | DaraFab-peptideNCIT | Δ(CIT-NCIT) (%) |
|---|---|---|---|---|
| IgG | 21.9 | 21.4 | 22.6 | -5% |
| | 24 | 24.6 | 23.4 | +5% |
| | 9.57 | 9.9 | 9.45 | +4% |
| | 26.2 | 25.1 | 27.3 | -8% |
| ACPA | 28.9 | 32.6 | 28.5 | +14% |
| | 26.4 | 29 | 25.8 | +12% |
| | 26.8 | 31.6 | 26.1 | +21% |
| | 27.9 | 32.4 | 27.1 | +19% |

Les résultats montrent une augmentation de la mortalité cellulaire en présence des ACPA lorsque les cellules DAUDI ont été traitées par l'hybride DaraFab-β60-74CIT par rapport à celles qui l'ont été par le DaraFab-β60-74NCIT. L'augmentation de la mort cellulaire en présence de la molécule hybride citrullinée (DaraFab-β60-74CIT) par rapport à la forme non citrullinée (DaraFab-β60-74NCIT) après pontage par les ACPA, est en moyenne de 16,5%.

Des cellules issues de la lignée Daudi (CD38+) ont été déposées en plaque 96 puits à raison de 50.000 cellules/puits en milieu RPMI complet (10% SVF, 1% glutamine, 1% pénicilline/streptomycine) et incubées durant 1h à 37°C avec 10µg/mL des hybrides DaraFab-β60-74CIT ou DaraFab-β60-74NCIT. Après lavage, ont été ajoutées des IgG humaines (contrôle négatif) ou des ACPA anti-β60-74 purifiés élués à pH3 à 5µg/mL, incubés durant 20h à 37°C. Un test de viabilité cellulaire (MTT : marqueur colorimétrique de l'activité mitochondriale cellulaire) a été réalisé. Les cellules ont ensuite été prélevées et la DO analysée par spectrométrie (Thermofisher Multiskan Fc). Les valeurs représentées correspondent aux DO obtenues dans chaque condition expérimentale.
Δ(CIT-NCIT) (%) représente la différence entre la DO obtenue en présence de DaraFab-β60-74CIT versus NCIT, divisée par la DO obtenue en présence de DaraFab-β60-74NCIT.

Les résultats sont présentés dans le Tableau 3.

**[Tableau 3]. Tableau 3 représente l'évaluation de la viabilité cellulaire des cellules de la lignée Daudi (CD38+) revêtues du DaraFab-β60-74Cit après pontage par les ACPAs purifiés élués à pH3. Synthèse de 3 expériences indépendantes.**

| Anticorps | Non traité | DaraFab-peptideCIT | DaraFab-peptideNCIT | Δ(CIT-NCIT) (%) |
|---|---|---|---|---|
| IgG | 0.716 | 0.717 | 0.696 | - 3% |
| | 0.886 | 0.825 | 0.792 | - 4% |
| | 1.055 | 1.018 | 0.996 | - 2% |
| ACPA | 0.571 | 0.497 | 0.589 | - 16% |
| | 0.61 | 0.566 | 0.721 | - 21% |
| | 0.822 | 0.702 | 0.829 | - 15% |

Les résultats montrent une diminution de la viabilité cellulaire en présence des ACPA lorsque les cellules Daudi ont été traitées par l'hybride DaraFab-β60-74CIT comparées à celles qui l'ont été par le DaraFab-β60-74NCIT.

La diminution de la viabilité cellulaire en présence de la molécule hybride citrullinée (DaraFab-β60-74CIT) par rapport à la forme non citrullinée (DaraFab-β60-74NCIT) après pontage par les ACPA, est en moyenne de 17,33%.

### Exemple 4 : Cytotoxicité du DaraFab-PEG-β60-74Cit vis-à-vis de la lignée B BAEV (BC-9) produite à partir de lymphocytes d'un patient atteint de polyarthrite rhumatoïde, après pontage par des ACPA anti-β60-74 purifiés

Les ACPA sont obtenus comme dans l'Exemple 2.

Des cellules de la lignée BC-9 (B BAEV) issues des lymphocytes sanguins d'un patient atteint de polyarthrite rhumatoïde ont été déposées en plaques de 96 puits à raison de 100.000 cellules/puits en milieu IMDM complet (10% SVF, 1% glutamine, 1% pénicilline/streptomycine) et incubées durant 1h à 37°C avec 10µg/mL des hybrides DaraFab-PEG-β60-74cit et DaraFab-PEG-β60-74arg. Après lavage ont été ajoutés des ACPA anti-β60-74 purifiés élués à pH3, à 5µg/mL durant 20h à 37°C, avant de procéder à un marquage par le 7-AAD (marqueur d'apoptose tardive). Les cellules ont ensuite été prélevées et analysées en cytométrie en flux (BD Biosciences, CANTO II). Les données présentées correspondant aux moyennes de 4 expériences indépendantes. L'analyse statistique a été effectuée selon un test de Student (t-test ; *p<0,05).

DaraFab-PEG-β60-74cit ici représente un Fab capable de se lier à CD38 lié à un espaceur PEGn, lui-même couplé à azoture qui est lié de façon covalente à un cyclooctyne (DBCO) qui est couplé à un peptide de SEQ ID NO : 19. DaraFab-PEG-β60-74arg ici représente un Fab capable de se lier à CD38 lié à un espaceur PEGn, lui-même couplé à un azoture qui est lié de façon covalente à un cyclooctyne (DBCO) qui est couplé à un peptide de SEQ ID NO : 19 dans laquelle les résidus arginyl n'ont pas été substitués par des résidus citrullyl (i.e. le peptide n'est pas citrulliné). n représente un nombre entre 1 et 10, de préférence 1, 2, 3, 4 ou 8. Les résultats (présentés en Figure 5) montrent ainsi qu'il Il existe une diminution significative de la viabilité cellulaire (partie gauche) en présence des ACPA purifiés lorsque les cellules BC-9 ont été préalablement incubées avec l'hybride citrulliné DaraFab-PEG-β60-74cit par rapport à la viabilité de celles qui l'ont été avec le DaraFab-PEG-β60-74arg. Parallèlement il existe une augmentation significative de l'apoptose tardive (partie droite) dans ces mêmes conditions.

### Exemple 5 : Cytotoxicité des hybrides Dara-Fab peptide-β60Cit et Dara-Fab peptide-α621Cit, vis-à-vis d'une lignée B BAEV différenciée, issue de lymphocytes d'un patient atteint de polyarthrite rhumatoïde), après pontage des hybrides par les ACPA anti-β60-74 purifiés élués à pH3

Les ACPA sont obtenus comme dans l'Exemple 2.

Des cellules issues de la lignée BC-9 (B BAEV) ont été différenciées (pendant 7 jours) afin d'augmenter l'expression du marqueur CD38 à leur surface comme montré dans la Figure 6.

DaraFab-PEG-α621-635 représente ici un fragment Fab capable de se lier à CD38 lié à un espaceur PEGn, lui-même couplé à un azoture qui est lié de façon covalente à un cyclooctyne (DBCO) qui est couplé à un espaceur PEGn qui est lui-même lié à un peptide de SEQ ID NO : 18. DaraFab-PEG-α621-635cit représente l'hybride dans lequel le peptide est citrulliné et DaraFab-PEG-α621-635arg représente le cas où le peptide n'est pas citrulliné. n représente un nombre entre 1 et 10, de préférence 1, 2, 3, 4 ou 8.

DaraFab-PEG-β60-74 représente ici un Fab capable de se lier à CD38 lié à un espaceur PEGn, lui-même couplé à un azoture qui est lié de façon covalente à un cyclooctyne (DBCO) qui est couplé à un espaceur PEGn qui est lui-même lié à un peptide de SEQ ID NO : 19. DaraFab-PEG-β60-74cit représente l'hybride dans lequel le peptide est citrulliné et DaraFab-PEG-β60-74 arg représente le cas où le peptide n'est pas citrulliné. n représente un nombre entre 1 et 10, de préférence 1, 2, 3, 4 ou 8.

Des cellules issues de la lignée BC-9 (B BAEV) produites à partir de lymphocytes sanguins d'un patient atteint de polyarthrite rhumatoïde soumises pendant 7 jours à un protocole de différenciation plasmocytaire, ont été déposées en plaques de 96 puits à raison de 100.000 cellules/puits en milieu IMDM complet (10% SVF, 1% glutamine, 1% pénicilline/streptomycine) puis incubées durant 30 minutes à 37°C avec 10µg/mL des hybrides DaraFab-PEG-β60-74cit/DaraFab-PEG-β60-74arg et DaraFab-PEG-α621-635cit/DaraFab-PEG-α621-635arg. Après lavage, ont été ajoutées des IgG humaines (contrôle négatif) ou des ACPA anti-β60-74 purifiés élués à pH3, à 5µg/mL durant 24hou 48h à 37°C, avant de procéder à un marquage par l'Annexine-V (marqueur d'apoptose précoce) et le 7-AAD (marqueur d'apoptose tardive). Les cellules ont ensuite été prélevées et analysées par un analyseur de mort cellulaire (Luminex, MUSE). Les valeurs montrées correspondent au pourcentage de cellules Annexine-V-positives. Δ(cit-arg) (%) représente la différence entre le pourcentage de cellules Annexine-V-positives en présence de DaraFab-PEG-β60-74cit ou DaraFab-PEG-α621-635cit et le pourcentage de cellules Annexine-V-positives en présence de DaraFab-PEG-β60-74arg ou DaraFab-PEG-α621-635arg, divisé par le pourcentage de cellules Annexine-V-positives en présence de DaraFab-PEG-β60-74arg ou DaraFab-PEG-α621-635arg.

Les résultats sont présentés dans le Tableau 4.

**[Tableau 4]. Tableau 4 représente la quantification de l'apoptose**

| | 24h | | | | 48h | | | |
|---|---|---|---|---|---|---|---|---|
| | DaraFab-PEG-α621-635 | | DaraFab-PEG-β60-74 | | DaraFab-PEG-α621-635 | | DaraFab-PEG-β60-74 | |
| | cit | | arg | | cit | | arg | |
| +IgG | 19% | 18% | 18% | 17% | 27% | 24% | 26% | 25% |
| +ACPA pH3 | 28% | 22% | 23% | 17% | 41% | 29% | 33% | 24% |
| Δcit-arg | +30% | | +32% | | +40% | | +41% | |

Les résultats indiquent la quantification de l'apoptose induite en présence des ACPA lorsque les cellules BC-9 ont été pré-traitées par le DaraFab-PEG-β60-74cit ou le DaraFab-PEG-α621-635cit, par rapport à celles qui l'ont été avec les formes non citrullinés DaraFab-PEG-β60-74arg et DaraFab-PEG-α621-635arg.

L'augmentation de l'apoptose en présence des molécules hybrides citrullinées (DaraFab-β60-74cit et DaraFab-PEG-α621cit par rapport aux formes non citrullinées, après pontage par les ACPA purifiés, est autour de 30% après 24h et de 40%, après 48h.

### Exemple 6 : Interaction de l'hybride DaraFab-PEG-α621-635 et des ACPA purifiés anti-α621-635cit ou de l'ACPA 2H06, à la surface de la lignée KMS-12-PE issue d'un myélome ou plasmocytome malin humain

Les ACPA (ici, ACPA purifiés) sont des ACPA obtenus à partir de sérum de patients atteints de polyarthrite rhumatoïde ACPA-positifs. De tels ACPA ont été obtenus par une méthode classique de chromatographie d'affinité sur colonne, connue de l'homme de l'art, utilisant comme antigènes liés, un ou plusieurs des cinq peptides immunodominants de l'invention (cf SEQ ID NO : 5, SEQ ID NO : 6, SEQ ID NO : 14, SEQ ID NO : 18 ou SEQ ID NO : 19).

L'ACPA ici également utilisé est un ACPA humain monoclonal recombinant ((clone 022014CCP14CFCT2H06, dit 2H06). Un tel ACPA peut être obtenu comme décrit dans Titcombe P. J., Wigerblad G., Sippl N., Zhang N., Shmagel A. K., Sahlström P., Zhang Y., Barsness L. O., Ghodke-Puranik Y., Baharpoor A., et al. Pathogenic Citrulline-Multispecific B Cell Receptor Clades in Rheumatoid Arthritis. Arthritis & Rheumatology 2018, 70 (12), 1933-1945. https://doi.org/10.1002/art.40590. Le VH de l'ACPA 2H06 a le numéro d'accession MH629710.1 sous Genbank, et le VL a le numéro d'accession MH629700.1.

Les hybrides DaraFab-PEG-α621-635 Cit/Arg ont été marqués avec des fluorochromes AlexaFluor^{®}647 et les ACPA purifiés anti-α621-635cit ainsi que l'ACPA 2H06 ont été marqués avec des fluorochromes AlexaFluor^{®}488 à l'aide de kits commerciaux (Lightning Link, Abcam). Des cellules issues de la lignée cellulaire myélomateuse humaine KMS-12-PE ont été déposées en plaque de 96 puits, à raison de 200.000 cellules/puits en milieu RPMI complet (10% SVF, 1% glutamine, 1% pénicilline/streptomycine) et incubées durant 30 minutes à 4°C avec 10µg/mL des hybrides DaraFab-PEG-α621-635 Cit/Arg marqués AlexaFluor^{®}647 puis incubées avec les ACPA purifiés anti-α621-635cit ou avec l'ACPA 2H06, marqués AlexaFluor^{®}488 pendant 30 minutes à 4°C à 5µg/mL. Après lavage dans un tampon PBS BSA 2%, les cellules KMS-12-PE ont été prélevées et analysées par cytométrie en flux (BD Biosciences, Canto II).

Les résultats présentés en Figure 7 montrent une interaction des molécules hybrides DaraFab-PEG-α621-635 Cit et des ACPA anti-α621-635cit ou de l'ACPA 2H06, à la surface de la lignée myélomateuse KMS-12-PE, visualisée par l'apparition d'une population double-positive AF647/AF488. Cette interaction est spécifique et liée au peptide car elle n'est pas observée en présence des hybrides DaraFab-PEG-α621-635 Arg.

### Exemple 7 : Phagocytose de la lignée myélomateuse différenciée KMS-12-PE par des macrophages, en présence des DaraFab-PEG-α621-635 et de l'ACPA 2H06

L'ACPA 2H06 est obtenu comme dans l'Exemple 6.
(**A**) Des cellules issues de la lignée myélomateuse KMS-12-PE ont été différenciées pendant 4 jours afin d'augmenter l'expression du marqueur CD38 à leur surface, à l'aide d'un mélange de cytokines composé d'IFN-α à 100ng/mL, d'IL-6 à 50ng/mL, et d'IL-15 à 10ng/mL. Les cellules ont ensuite été marquées au CFSE (Carboxyfluorescein Succinimidyl Ester, marqueur intracellulaire fluorescent) et mises en présence de Daratumumab (AC monoclonal anti-CD38 utilisé comme contrôle positif) ou en présence des hybrides DaraFab-PEG-α621-635 Cit/Arg durant 30 minutes à 10µg/mL en milieu RPMI complet à 37°C. Puis, les cellules ont été incubées avec l'ACPA 2H06 à 50µg/mL pendant 30 minutes en milieu RPMI complet à 37°C et mises en contact, sans lavage, avec des macrophages humains (différenciés in vitro en présence de M-CSF (100ng/mL) à partir de monocytes CD14+ isolés du sang périphérique d'un sujet sain, non marqués), à raison de 1 cellule KMS-12-PE pour 1 macrophage, durant 2h à 37°C. Les macrophages ont ensuite été décollés puis analysés par cytométrie en flux à l'aide d'un marquage Cd11b BV421 (marqueur membranaire spécifique des macrophages). Les différentes populations cellulaires sont exprimées en pourcentage de la population cellulaire totale et comprennent le pourcentage de doubles-positives CFSE/BV421 (macrophages qui ont phagocyté des cellules KMS-12-PE) et le pourcentage de la population-cible KMS-12-PE résiduelle. (**B**) Les données sont représentatives de 4 expériences identiques indépendantes. L'analyse statistique a été effectuée selon un test de Student apparié (paired t-test ; **p<0,01 ; ***p<0.001).
(**A**) Les résultats présentés en Figure 8 montrent que l'activité de phagocytose est majorée en présence des hybrides DaraFab-PEG-α625-631 Cit et de l'ACPA 2H06. Une augmentation des événements doubles-positifs de 70% est observée et associée à une diminution de 73% de la population-cible. De plus, l'activité de phagocytose obtenue en présence des hybrides DaraFab-PEG-α625-631 Cit et de l'ACPA 2H06 est plus élevée que celles obtenue avec le contrôle Daratumumab. (**B**) Cette expérience a été réalisée quatre fois et une augmentation spécifique et significative (***p<0,001, paired t-test) de 78% de la population double-positive a été observée en présence des hybrides DaraFab-PEG-α625-631 Cit et de l'ACPA 2H06, ainsi qu'une diminution significative (**p<0,01, paired t-test) de 75% de la population-cible.

### Exemple 8 : Phagocytose de la lignée myélomateuse différenciée KMS-12-PE par des macrophages en présence de DaraFab-PEG-β60-74 ou de DaraFab-PEG-α621-635 et d'ACPA purifiés, respectivement anti-β60-74cit ou anti-α621-635cit

L'ACPA 2H06 est obtenu comme dans l'Exemple 6.

Des cellules issues de la lignée myélomateuse KMS-12-PE ont été différenciées pendant 4 jours afin d'augmenter l'expression du marqueur CD38 à leur surface, à l'aide d'un mélange de cytokines composé d'IFN-α à 100ng/mL, d'IL-6 à 50ng/mL, et d'IL-15 à 10ng/mL. Les cellules ont ensuite été marquées au CFSE (Carboxyfluorescein Succinimidyl Ester, marqueur intracellulaire fluorescent) et mises en présence de Daratumumab (anticorps monoclonal anti-CD38 utilisé comme contrôle positif) ou en présence des hybrides DaraFab-PEG-α621-635 Cit/Arg ou des hybrides DaraFab-α621-635 Cit/Arg ou des hybrides DaraFab-PEG-β60-74 Cit/Arg ou des hybrides DaraFab- β60-74 Cit/Arg, durant 30 minutes à 10µg/mL en milieu RPMI complet à 37°C. Puis, les cellules ont été incubées avec les ACPAs purifiés anti-α621-635cit ou anti-β60-74cit à 5µg/mL pendant 30min en milieu RPMI complet à 37°C. Les cellules KMS-12-PE sont mises en contact, sans lavage, avec des macrophages humains, différenciés *in vitro* en présence de M-CSF (100ng/mL), à partir de monocytes CD14+ isolés du sang périphérique d'un sujet sain, non marqués, à raison de 1 cellule KMS-12-PE pour 1 macrophage, durant 2h à 37°C. Les macrophages ont ensuite été décollés puis analysés par cytométrie en flux à l'aide d'un marquage Cd11b BV421 (marqueur membranaire spécifique des macrophages).

Les résultats sont présentés en Figure 9 et montrent que l'activité de phagocytose est majorée en présence des hybrides DaraFab-peptide α625-631 Cit ou DaraFab-peptide β60-74 Cit et des ACPAs purifiés correspondants, anti-α621-635cit ou anti-β60-74cit. De plus, l'activité de phagocytose obtenue en présence des hybrides DaraFab-peptide α625-631 Cit et des ACPAs purifiés anti-α625-631cit, est plus élevée que celles obtenue avec le contrôle Daratumumab. Dans les conditions utilisant les hybrides DarFabβ60-74cit et les ACPAs purifiés anti-β60-74cit, l'activité de phagocytose est équivalente à celle obtenue avec le contrôle Daratumumab.

### Exemple 9 : Induction de l'apoptose des cellules de la lignée myélomateuse différenciée KMS-12-PE par des cellules NK, en présence de DaraFab-PEG-α621-635 et d'ACPA purifiés anti-α621-635cit ou d'ACPA 2H06

Les ACPA purifiés et l'anticorps monoclonal 2H06 sont obtenus comme décrit dans l'Exemple 6.

Des cellules issues de la lignée myélomateuse KMS-12-PE ont été différenciées pendant 3 jours afin d'augmenter l'expression du marqueur CD38 à leur surface, à l'aide d'un mélange de cytokines comprenant de l'IFN-α à 100ng/mL, de l'IL-6 à 50ng/mL, et de l'IL-15 à 10ng/mL. Dans une plaque de 96 puits, 100.000 cellules/puits ont été incubées avec les molécules hybrides DaraFab-α621-635 Cit pendant 30min en milieu RPMI complet à 37°C à 10µg/mL puis avec les ACPA 2H06 à 50µg/mL ou avec les ACPAs purifiés anti-α621-635cit à 10µg/mL, en présence de cellules NK fraichement purifiées (marquées avec le Cell Tracker Violet) utilisées comme cellules effectrices, également à 100.000 cellules/puits, durant 16h à 37°C. Les molécules hybrides DaraFab- α621-635 Arg, utilisées comme contrôles de spécificité, ont été incubées dans les mêmes conditions. Comme contrôles négatifs, les cellules KMS-12-PE ont été incubées en l'absence d'hybrides, seules ou en présence de cellules NK. La lyse cellulaire a été mesurée en cytométrie de flux à l'aide d'un marquage avec les sondes fluorescentes 7-AAD et Annexine-V-PE. Les résultats sont présentés en Figure 10 et montrent que les hybrides DaraFab-PEG-α621-635 Cit, en présence des ACPA purifiés anti-α621-635cit ou des ACPA 2H06, sont capables de majorer spécifiquement la lyse par ADCC des cellules-cibles KMS-12-PE provoquée par les cellules NK. La destruction des cellules-cibles est augmentée de 47% à 68% en présence des ACPA 2H06 et de 49% à 57% en présence des ACPA purifiés anti-α621-635cit.

## Revendications

1. Molécule hybride comprenant au moins un peptide dérivé de fibrine ayant au moins un résidu citrullyl, ledit peptide étant lié de façon covalente à au moins un anticorps ou à au moins un fragment d'anticorps, ledit anticorps ou fragment étant capable de se lier à CD38 et/ou CD138, un ou plusieurs espaceurs étant optionnellement présents entre ledit peptide et ledit anticorps ou ledit fragment, ledit peptide étant reconnu par les auto-anticorps anti-protéines citrullinées et étant dérivé de tout ou partie de la séquence de la chaine α ou β d'une fibrine de vertébré, par substitution d'au moins un résidu arginyl par un résidu citrullyl.

2. Molécule hybride selon la revendication 1, dans laquelle ledit peptide est lié de façon covalente à un espaceur, ledit espaceur étant lui-même lié de façon covalente audit anticorps ou fragment d'anticorps.

3. Molécule hybride selon l'une quelconque des revendications précédentes, ladite fibrine de vertébré étant une fibrine de mammifère, de préférence humaine.

4. Molécule hybride selon l'une quelconque des revendications précédentes, dans laquelle ledit espaceur est un polymère contenant un ou plusieurs motifs de répétition contenant le groupe éther, ledit espaceur étant de préférence le polyéthylène glycol de formule PEGn, dans laquelle n représente un nombre entier compris entre 1 et 100, préférentiellement entre 1 et 10 et notamment 1, 2, 3, 4 ou 8.

5. Molécule hybride selon l'une quelconque des revendications précédentes, dans laquelle ledit peptide comprend au moins un résidu citrullyl, et est choisi dans le groupe constitué par :
a) un peptide défini par la séquence X₁PAPPPISGGGYX₂AX₃ (SEQ ID NO : 1) dans laquelle X₁, X₂, et X₃ représentent chacun un résidu citrullyl ou un résidu arginyl, et au moins l'un des résidus X1 ou X₂ ou X₃ est un résidu citrullyl ;
b) un peptide défini par la séquence GPX₁VVEX₂HQSACKDS (SEQ ID NO : 2) dans laquelle X₁ et X₂ représentent chacun un résidu citrullyl ou un résidu arginyl, et au moins l'un des résidus X₁ ou X₂ est un résidu citrullyl ;
c) un peptide défini par la séquence SGIGTLOGFX₁HX₂HPO (SEQ ID NO : 3) dans laquelle X₁ et X₂ représentent chacun un résidu citrullyl ou un résidu arginyl, et au moins l'un des résidus X₁ ou X₂ est un résidu citrullyl ;
d) un peptide défini par la séquence VDIDIKIX₁SCX₂GSCS (SEQ ID NO : 4) dans laquelle X₁ et X₂ représentent chacun un résidu citrullyl ou un résidu arginyl, et au moins l'un des résidus X₁ ou X₂ est un résidu citrullyl ;
e) un peptide défini par la séquence X₁GHAKSX₂PVX₃GIHTS (SEQ ID NO : 12) dans laquelle X₁, X₂ et X₃ représentent chacun un résidu citrullyl ou un résidu arginyl, et au moins l'un des résidus X₁ ou X₂ ou X₃ est un résidu citrullyl ;
f) un peptide comprenant au moins 5 acides aminés consécutifs, dont au moins un résidu citrullyl, de l'un des peptides a) à e) ci-dessus.

6. Molécule hybride selon l'une quelconque des revendications 1-4, dans laquelle ledit peptide est choisi dans le groupe constitué par : SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22 et SEQ ID NO : 23, plus particulièrement choisi dans le groupe constitué par : SEQ ID NO : 5, SEQ ID NO : 6, SEQ ID NO : 14, SEQ ID NO : 18 et SEQ ID NO : 19, encore particulièrement SEQ ID NO : 18 et SEQ ID NO : 19.

7. Molécule hybride selon l'une quelconque des revendications précédentes, dans laquelle le fragment est choisi parmi les fragments, scFc, Fv, Fab ou F(ab')₂.

8. Molécule hybride selon l'une quelconque des revendications précédentes, dans laquelle ledit anticorps ou fragment F(ab')₂ est un anticorps ou fragment F(ab')₂ bispécifique, dirigé contre :
- CD38 et une autre cible plasmocytaire, ou
- CD138 et une autre cible plasmocytaire, ou
- CD38 et CD138.

9. Molécule hybride selon l'une quelconque des revendications précédentes, ladite molécule étant représentée par :
- un fragment Fab d'anticorps capable de se lier à CD38 lié à un espaceur PEGn, lui-même couplé à un azoture lié de façon covalente à un cyclooctyne couplé à un peptide représenté par SEQ ID NO : 19, ou SEQ ID NO : 18,
- un fragment Fab d'anticorps capable de se lier à CD38 lié à un espaceur PEGn, lui-même couplé à un azoture lié de façon covalente à un cyclooctyne couplé à un espaceur PEGn, lui-même lié à un peptide représenté par SEQ ID NO : 19 ou SEQ ID NO : 18,
n représentant de préférence un entier entre 1 et 10, plus particulièrement 1, 2, 3, 4 ou 8.

10. Molécule hybride selon l'une quelconque des revendications précédentes, pour son utilisation comme médicament, notamment pour son utilisation dans le traitement des maladies auto-immunes associées à la production d'auto-anticorps anti-protéines citrullinées, en particulier le syndrome de Gougerot-Sjögren et la polyarthrite rhumatoïde.

11. Molécule hybride selon l'une quelconque des revendications précédentes, pour son utilisation comme médicament, en combinaison avec une deuxième molécule hybride, ladite deuxième molécule comprenant au moins un fragment Fc d'anticorps lié de façon covalente à au moins un peptide dérivé de fibrine porteur d'un résidu citrullyl, un espaceur étant optionnellement présent entre ledit fragment Fc et ledit peptide.

## Patentansprüche

1. Hybridmolekül, umfassend mindestens ein aus Fibrin abgeleitetes Peptid, das mindestens einen Citrullinrest aufweist, wobei das Peptid kovalent an mindestens einen Antikörper oder ein Antikörperfragment gebunden ist, wobei der Antikörper oder das Fragment in der Lage ist, an CD38 und/oder CD138 zu binden, wobei zwischen dem Peptid und dem Antikörper oder dem Fragment gegebenenfalls ein oder mehrere Spacer vorliegen, wobei das Peptid von den Anti-citrulliniertes Protein-AutoAntikörpern erkannt wird und von der gesamten oder einem Teil der Sequenz der α- oder β-Kette eines Vertebratenfibrins durch Substitution mindestens eines Arginylrests duch einen Citrulinrest abgeleitet ist.

2. Hybridmolekül nach Anspruch 1, wobei das Peptid kovalent an einen Spacer gebunden ist, wobei der Spacer seinerseits kovalent an den Antikörper oder das Fragment gebunden ist.

3. Hybridmolekül nach einem der vorangehenden Ansprüche, wobei das Vertebratenfibrin ein Säugerfibrin ist, bevorzugt humanes Fibrin.

4. Hybridmolekül nach einem der vorangehenden Ansprüche, wobei der Spacer ein Polymer ist, das ein oder mehrere Wiederholungseinheiten enthält, die eine Ethergruppe enthält/enthalten, wobei der Spacer bevorzugt Polyethylenglycol der Formel PEGn ist, wobei n eine ganze Zahl zwischen 1 und 100, bevorzugt zwischen 1 und 10, und insbesondere 1, 2, 3, 4 oder 8 ist.

5. Hybridmolekül nach einem der vorangehenden Ansprüche, wobei das Peptid mindestens einen Citrullinrest umfasst und ausgewählt ist aus der Gruppe bestehend aus:
a) einem Peptid, das durch die Sequenz X₁PAPPPISGGGYX₂AX₃ (SEQ ID NO:1) definiert ist, wobei X₁, X₂ und X₃ jeweils einen Citrullinrest oder einen Arginylrest darstellen und mindestens einer der Reste X₁ oder X₂ oder X₃ ein Citrullinrest ist;
b) einem Peptid, das durch die Sequenz GPX₁VVEX₂HQSACKDS (SEQ ID NO:2) definiert ist, wobei X₁ und X₂ jeweils einen Citrullinrest oder einen Arginylrest darstellen und mindestens einer der Reste X₁ oder X₂ ein Citrullinrest ist;
c) einem Peptid, das durch die Sequenz SGIGTLDGFX₁HX₂HPD (SEQ ID NO:3) definiert ist, wobei X₁ und X₂ jeweils einen Citrullinrest oder einen Arginylrest darstellen und mindestens einer der Reste X₁ oder X₂ ein Citrullinrest ist;
d) einem Peptid, das durch die Sequenz VDIDIKIX₁SCX₂GSCS (SEQ ID NO:4) definiert ist, wobei X₁ und X₂ jeweils einen Citrullinrest oder einen Arginylrest darstellen und mindestens einer der Reste X₁ oder X₂ ein Citrullinrest ist;
e) einem Peptid, das durch die Sequenz X₁GHAKSX₂PVX₃GIHTS (SEQ ID NO:12) definiert ist, wobei X₁, X₂ und X₃ jeweils einen Citrullinrest oder einen Arginylrest darstellen und mindestens einer der Reste X₁ oder X₂ oder X₃ ein Citrullinrest ist;
f) einem Peptid, das mindestens 5 aufeinanderfolgende Aminosäuren, darunter mindestens einen Citrullinrest, aus einem der oben genannten Peptide a) bis e) umfasst.

6. Hybridmolekül nach einem der Ansprüche 1 bis 4, wobei das Peptid ausgewählt ist aus der Gruppe bestehend aus: SEQ ID NO:5, SEQ ID NO:6, SEQ ID NO:7, SEQ ID NO:8, SEQ ID NO:9, SEQ ID NO:10, SEQ ID NO:11, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22 und SEQ ID NO:23, stärker bevorzugt ausgewählt aus der Gruppe bestehend aus: SEQ ID NO:5, SEQ ID NO:6, SEQ ID NO:14, SEQ ID NO:18 und SEQ ID NO:19, noch stärker bevorzugt SEQ ID NO:18 und SEQ ID NO:19.

7. Hybridmolekül nach einem der vorangehenden Ansprüche, wobei das Fragment ausgewählt ist aus den Fragmenten scFc, Fv, Fab oder F(ab')₂.

8. Hybridmolekül nach einem der vorangehenden Ansprüche, wobei der Antikörper oder das Fragment F(ab')₂ ein bispezfischer Antikörper oder ein bispezifisches Fragment F(ab')₂ ist, das gerichtet ist gegen:
- CD38 und ein weiteres Plasmocytenziel, oder
- CD138 und ein weiteres Plasmocytenziel, oder
- CD38 und CD138.

9. Hybridmolekül nach einem der vorangehenden Ansprüche, wobei das Molekül dargestellt ist durch:
- ein Fab-Fragment eines Antikörpers, das in der Lage ist, an CD38, das an einen PEGn-Spacer gebunden ist, zu binden, der seinerseits an ein Azid gebunden ist, das kovalent an ein Cyclooctyn gebunden ist, das an ein durch SEQ ID NO:19 oder SEQ ID NO:18 dargestelltes Peptid gebunden ist;
- ein Fab-Fragment eines Antikörpers, das in der Lage ist, an CD38, das an einen PEGn-Spacer gebunden ist, zu binden, der seinerseits an ein Azid gebunden ist, das kovalent an ein Cyclooctyn gebunden ist, das an einen PEGn-Spacer gebunden ist, der seinerseits an ein durch SEQ ID NO:19 oder SEQ ID NO:18 dargestelltes Peptid gebunden ist;
wobei n bevorzugt eine ganze Zahl zwischen 1 und 10, stärker bevorzugt 1, 2, 3, 4 oder 8 ist.

10. Hybridmolekül nach einem der vorangehenden Ansprüche zur Verwendung als Medikament, insbesondere zur Verwendung bei der Behandlung von Autoimmunerkrankungen, die mit der Produktion von Anti-citrulliniertes Protein-Auto-Antikörpern im Zusammenhang stehen, insbesondere des Gougerot-Sjögren-Syndroms und rheumatoider Arthritis.

11. Hybridmolekül nach einem der vorangehenden Ansprüche zur Verwendung als Medikament in Kombination mit einem zweiten Hybridmolekül, wobei das zweite Molekül mindestens ein Fc-Fragment eines Antikörpers umfasst, das kovalent an mindestens ein von Fibrin abgeleitetes Peptid, das einen Citrullinrest trägt, gebunden ist, wobei gegebenenfalls zwischen dem Fc-Fragment und dem Peptid ein Spacer vorliegt.

## Claims

1. A hybrid molecule comprising at least one fibrin-derived peptide having at least one citrullyl residue, said peptide being covalently bound to at least one antibody or to at least one antibody fragment, said antibody or fragment being capable of binding to CD38 and/or CD138, one or more spacers being optionally present between said peptide and said antibody or said fragment, said peptide being recognised by the anti-citrullinated protein autoantibodies and being derived from all or part of the sequence of the α or β chain of a vertebrate fibrin by substitution of at least one arginyl residue by a citrullyl residue.

2. A hybrid molecule according to claim 1, wherein said peptide is covalently bound to a spacer, said spacer being itself covalently bound to said antibody or antibody fragment.

3. A hybrid molecule according to either one of the preceding claims, said vertebrate fibrin being a mammalian fibrin, preferably a human fibrin.

4. A hybrid molecule according to any one of the preceding claims, wherein said spacer is a polymer containing one or more repeat units containing the ether group, said spacer preferably being polyethylene glycol of formula PEGn, in which n represents an integer between 1 and 100, preferably between 1 and 10 and in particular 1, 2, 3, 4 or 8.

5. A hybrid molecule according to any one of the preceding claims, wherein said peptide comprises at least one citrullyl residue, and is selected from the group consisting of:
a) a peptide defined by the sequence X₁PAPPPISGGGYX₂AX₃ (SEQ ID NO: 1) in which X₁, X₂, and X₃ each represent a citrullyl residue or an arginyl residue, and at least one of the X₁ or X₂ or X₃ residues is a citrullyl residue;
b) a peptide defined by the sequence GPX₁VVEX₂HQSACKDS (SEQ ID NO: 2) in which X₁ and X₂ each represent a citrullyl residue or an arginyl residue, and at least one of the X₁ or X₂ residues is a citrullyl residue;
c) a peptide defined by the sequence SGIGTLDGFX₁HX₂HPD (SEQ ID NO:3) in which X₁ and X₂ each represent a citrullyl residue or an arginyl residue, and at least one of the X₁ or X₂ residues is a citrullyl residue;
d) a peptide defined by the sequence VDIDIKIX₁SCX₂GSCS (SEQ ID NO:4) in which X₁ and X₂ each represent a citrullyl residue or an arginyl residue, and at least one of the X₁ or X₂ residues is a citrullyl residue;
e) a peptide defined by the sequence X₁GHAKSX₂PVX₃GIHTS (SEQ ID NO: 12) in which X₁, X₂ and X₃ each represent a citrullyl residue or an arginyl residue, and at least one of the X₁ or X₂ or X₃ residues is a citrullyl residue;
f) a peptide comprising at least 5 consecutive amino acids, including at least one citrullyl residue, from one of peptides a) to e) above.

6. A hybrid molecule according to any one of claims 1-4, wherein said peptide is selected from the group consisting of: SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22 and SEQ ID NO: 23, more particularly selected from the group consisting of: SEQ ID NO: 5, SEQ ID NO: 6, SEQ ID NO: 14, SEQ ID NO: 18 and SEQ ID NO: 19, more particularly SEQ ID NO: 18 and SEQ ID NO: 19.

7. A hybrid molecule according to any one of the preceding claims, wherein the fragment is selected from fragments, scFc, Fv, Fab or F(ab')₂.

8. A hybrid molecule according to any one of the preceding claims, wherein said antibody or F(ab')₂ fragment is a bispecific antibody or F(ab')₂ fragment, directed against:
- CD38 and another plasma cell target, or
- CD138 and another plasma cell target, or
- CD38 and CD138.

9. A hybrid molecule according to any one of the preceding claims, said molecule being represented by:
- an antibody fragment capable of binding to CD38 bound to a PEGn spacer, itself coupled to an azide covalently bound to a cyclooctyne coupled to a peptide represented by SEQ ID NO: 19, or SEQ ID NO: 18,
- an antibody fragment capable of binding to CD38 bound to a PEGn spacer, itself coupled to an azide covalently bound to a cyclooctyne coupled to a PEGn spacer, itself bound to a peptide represented by SEQ ID NO:19 or SEQ ID NO: 18,
n preferably representing an integer between 1 and 10, more particularly 1, 2, 3, 4 or 8.

10. A hybrid molecule according to any one of the preceding claims for use as a medicinal product, in particular for use in the treatment of autoimmune diseases associated with the production of anti-citrullinated protein autoantibodies, in particular Gougerot-Sjögren syndrome and rheumatoid arthritis.

11. A hybrid molecule according to any one of the preceding claims, for use as a medicinal product, in combination with a second hybrid molecule, said second molecule comprising at least one Fc antibody fragment covalently bound to at least one fibrin-derived peptide carrier of a citrullyl residue, a spacer being optionally present between said Fc fragment and said peptide.
